# EUROPEAN PATENT APPLICATION

(11) **EP 4 671 264 A1**
(43) Date of publication of application: **31.12.2025**
(21) Application number: 24185491.8
(22) Date of filing: 28.06.2024
(51) Int. Cl.: C07K 14/005, C12N 15/86, C12N 15/85

(54) **AAV PLASMIDS COMPRISING AN ACCESSORY PROTEIN CODING SEQUENCE**

(71) Applicant: Sartorius Stedim Cellca GmbH, 89081 Ulm (DE)
(72) Inventor: Jung, Ulrike, 89081 Ulm (DE)
(74) Representative: Cohausz & Florack

(57) **Abstract**

The invention relates to a rep plasmid comprising at least one adeno-associated virus replication protein coding sequence encoding at least one functional rep protein, and at least one accessory protein coding sequence, wherein the rep plasmid is not capable of encoding a functional cap protein.

## Description

### FIELD OF THE INVENTION

The present invention relates to a rep plasmid comprising at least one adeno-associated virus replication protein coding sequence encoding at least one functional rep protein, and at least one accessory protein coding sequence, wherein the rep plasmid is not capable of encoding a functional cap protein. The invention further concerns a plasmid system for producing an adeno-associated virus particle comprising the rep plasmid of the invention and a cap plasmid. In still another aspect of the invention, a cell comprising the rep plasmid of the invention, or the plasmid system of the invention is provided. In still another aspect of the invention, a kit comprising the expression system or cell is provided. In still another aspect, the invention provides a method of producing recombinant adeno-associated viral vectors comprising transfecting cells with the rep plasmid of the invention or plasmid system of the invention.

### BACKGROUND OF THE INVENTION

Several gene delivery techniques have been developed to express a gene of interest in cells, tissues, or organisms, including mammalian and in particular human cells, tissues, or organisms.

Some of these delivery techniques use a viral vector derived from lentivirus, oncoretrovirus, adenovirus, adeno-associated virus or similar viruses. Among these vectors, the adeno-associated virus (AAV) vector has been recognized as a promising tool. Adeno-associated viruses (AAVs) are linear, single-stranded DNA viruses that belong to the parvovirus family. AAVs are infectious to cells of a wide range of species, including humans, and can infect non-dividing cells in which differentiation has ceased, such as blood cells, muscle or nerve cells. In addition, wild-type AAVs are non-pathogenic to humans, and AAV particles are physiochemically very stable. These characteristics have led to the development of recombinant AAVs (rAAVs) as vectors for gene delivery and in particular gene therapy.

The single-stranded genome of wild-type AAV comprises rep (replication) and cap (capsid) genes. These genes give rise to several rep and cap proteins through alternative translation start sites and differential splicing. The coding sequences are flanked by inverted terminal repeats (ITRs), leading the AAV genome to form a T-shaped hairpin structure through the ITR at both ends, wherein the linear single-stranded genome between the hairpin structures encodes the rep and cap proteins. The rep gene encodes four proteins (rep78, rep68, rep52, and rep40) for viral genome replication and packaging, while cap expression gives rise to three capsid proteins (VP 1, VP2, and VP3), which form the outer capsid shell that protects the AAV genome, as well as being involved in cell binding and internalization.

A recombinant AAV (rAAV), which lacks the wild-type genome, is a protein-based nanoparticle designed to cross the cell membrane where it can transport and deliver its recombinant DNA cargo into the nucleus of a cell. In the absence of the rep proteins, ITR-flanked transgenes encoded by rAAV can form circular concatemers that persist as episomes in the nucleus of transduced cells. Because recombinant episomal DNA does not integrate into the host genome, it will eventually become diluted over time as the cell undergoes repeated rounds of replication. This will eventually lead to loss of the transgene and transgene expression, with the rate of transgene loss depending on the turnover rate of the transduced cell. These characteristics make rAAV ideal for gene therapy applications and AAV-vector-mediated gene delivery was recently approved for the treatment of inherited blindness and spinal muscular atrophy, and long-term therapeutic effects have been achieved for other rare diseases, including hemophilia and Duchenne muscular dystrophy.

Typical recombinant adeno-associated viral vectors (rAAV vectors) have a genome structure in which the rep and cap genes between the ITRs of the wildtype AAV genome are replaced by one or more transgenes to form a transgene plasmid for gene delivery in gene therapy applications. An example of the method of producing an rAAV vector is a method comprising introducing into a host cell a transgene plasmid in which a transgene is inserted between ITRs and introducing a rep plasmid for supplying the rep protein for replication to produce an rAAV vector in the host cell. The rep plasmid can further comprise the cap gene or the cap gene can be located on a separate cap plasmid introduced into the host cell. Furthermore, accessory helper genes from other viruses like Herpes or Adenoviruses can be introduced in the cell to regulate cell metabolism as well as AAV gene expression, prevent apoptosis, and other functions.

Manufacturing of rAAVs can use a plasmid system in which a single plasmid comprises the rep gene and the cap gene, ordinarily named "rep-cap plasmid". In the alternative, rAAVs can be manufactured using a first plasmid comprising the rep gene and a second plasmid comprising the cap gene, ordinarily named "rep/cap split plasmid system" or "trans-split rep-cap two-plasmid system". This rep/cap split plasmid system has the advantage of achieving a combination of improved economics resulting from the simpler and more flexible format, with enhanced safety and quality attributes in addition to other advantages. By using a rep/cap split plasmid system the capsid coding sequence(s) may be easily exchanged or altered in order to improve treatment of different genetic disorders, e.g., capsid variants may be designed and selected that enable potent delivery to desired tissues.

While wildtype AAV is capable of providing 100% full and infectious particles, with rAAV the range is usually with 1-30% full (the latter usually achieved rarely and only with very intense effort for particular transgenes and other elements combinations) and only 1 out of 200-1000 full particles infectious. Consequently, when using an rAAV vector for gene therapy applications, it is challenging to produce a sufficiently high titer required for efficient and effective treatment. The cause for the decreased titer is not completely understood. High levels of product-related impurities, e.g., empty/partially filled viral capsids, during production further challenge rAAV production. Product-related impurities can include AAV empty capsids, encapsidated host cell nucleic acid/helper DNA, and noninfectious AAV capsids. A purification process that cannot substantially remove this product-related impurity can result in exacerbated immune responses and reduced transduction efficiency. Independent of the improved economics, the rep/cap split plasmid system manufactured rAAV particles exhibit a further reduced potency compared to the single rep-cap plasmid, resulting in less efficient and effective treatment of genetic disorders.

Attempts to improve rAAV production when using the rep/cap split plasmid system include regulating the timing of cap expression to increase rAAV yield or altering the ratio of rep expression and cap expression for better AAV packaging (Ohba, Kenji et al. "Adeno-associated virus vector system controlling capsid expression improves viral quantity and quality." iScience vol. 26,4 106487. 23 Mar. 2023, doi:10.1016/j.isci.2023.106487, Ogasawara, Y et al. "Efficient production of adeno-associated virus vectors using split-type helper plasmids." Japanese journal of cancer research: Gann vol. 90,4 (1999): 476-83. doi: 10.1111/j.1349-7006.1999.tb00772.x).

In order to satisfy the demand of rAAV vectors for clinical trials and market supply, the following goals have yet to be achieved: (a) improved flexibility of the plasmid system for designing various gene therapies, (b) improved potency of rAAV vectors, (c) improved economics of rAAV manufacture, (d) high rAAV production yields; and (e) control over the proportion of produced rAAV particles containing ("full") or lacking ("empty") a complete recombinant vector genome.

### SUMMARY OF THE INVENTION

Against the aforementioned background, it is an object of the present invention to provide improved means for flexible rAAV vector production. It is also an object of the invention to provide improved rAAV vectors (or rAAV particles) capable of successfully transducing cells. It is a further object of the invention to provide rAAV vectors with improved transduction efficiency. In particular, it is an object of the present invention to provide rAAV vectors having an improved potency. It is thus an object of the invention to provide improved plasmid systems for rAAV production that enable production of high titers of rAAV particles with high potency and enable effective treatment of genetic disorders.

These objects are achieved by the invention set forth in the claims and embodiments explained in more detail below.

The invention provides a rep plasmid comprising:
(i) at least one adeno-associated virus replication protein coding sequence encoding at least one functional rep protein; and
(ii) at least one accessory protein coding sequence;
wherein the rep plasmid is not capable of encoding a functional cap protein.

The invention further concerns a plasmid system for producing an adeno-associated virus particle comprising:
(i) a cap plasmid comprises at least one adeno-associated virus capsid protein coding sequence encoding at least one functional capsid protein, and
(ii) the rep plasmid of the invention.

The invention further concerns a stable or transient cell expression system comprising the plasmid system of the invention.

The invention further concerns a kit comprising a stable or transient cell expression system of the invention or a cell of the invention, and a cell culture medium. This kit provides improved means for efficient rAAV vector production through the rep plasmid of the invention.

The invention further concerns a method for producing rAAV vectors by transfecting cells with the rep plasmid of the invention, or the plasmid system of the invention.

### DETAILED DESCRIPTION

Although certain embodiments of the present invention are described in detail below, it is to be understood that this invention is not limited to the particular embodiments, methodologies, protocols and reagents described herein as these may vary within the scope set by the claims. It is also to be understood that terminology used herein is for the purpose of describing particular embodiments only, and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The above objects are achieved by the following embodiments in accordance with the invention:
1. A rep plasmid comprising:
   (i) at least one adeno-associated virus replication protein coding sequence encoding at least one functional rep protein; and
   (ii) at least one accessory protein coding sequence;
   wherein the rep plasmid is not capable of encoding a functional cap protein.
2. The rep plasmid according to embodiment 1, wherein the rep plasmid comprises at least one replication protein coding sequence encoding at least two functional rep proteins, at least three functional rep proteins, or at least four functional rep proteins.
3. The rep plasmid according to embodiment 1, wherein the rep plasmid comprises at least two replication protein coding sequences, each encoding at least one functional rep protein, preferably wherein the rep plasmid comprises two replication protein coding sequences, each encoding one functional rep protein.
4. The rep plasmid according to any one of embodiments 1-3, wherein the replication protein coding sequence encodes one or more of rep68, rep78, rep52, and rep40 or artificial variants thereof.
5. The rep plasmid according to any one of embodiments 1-4, wherein the rep plasmid comprises a first replication protein coding sequence encoding the functional rep protein selected from rep68 and rep78 or artificial variants thereof, and a second replication protein coding sequence encoding the functional rep protein selected from rep52 and rep40 or artificial variants thereof.
6. The rep plasmid according to any one of embodiments 1-5, wherein the accessory protein coding sequence encodes one or more of AAP, MAAP, and X, or artificial variants thereof, preferably wherein the accessory protein coding sequence encodes at least MAAP or artificial variants thereof.
7. The rep plasmid according to embodiment 6, wherein the accessory protein coding sequence encodes MAAP or an artificial variant thereof.
8. The rep plasmid according to embodiment 6 or 7, wherein the accessory protein coding sequence encodes X or an artificial variant thereof.
9. The rep plasmid according to any one of embodiments 6-8, wherein the accessory protein coding sequence encodes AAP or an artificial variant thereof.
10. The rep plasmid according to any one of embodiments 1-9, wherein the rep plasmid comprises a first accessory protein coding sequence encoding MAAP or an artificial variant thereof and a second accessory protein coding sequence encoding X or an artificial variant thereof.
11. The rep plasmid according to any one of embodiments 1-10, wherein the replication protein coding sequence is from an AVV serotype selected from the group consisting of AAV-1, AAV-2, AAV-3, AAV-4, AAV-5, AAV-6, AAV-7, and AAV-8, preferably selected from AAV2.
12. The rep plasmid according to any one of embodiments 1-11, further comprising a native or heterologous promoter operably linked to the replication protein coding sequence, preferably wherein the promoter is selected from p5, p19, and p40 or an artificial promoter.
13. The rep plasmid according to any one of embodiments 5-12, further comprising a first native or heterologous promoter operably linked to the first replication protein coding sequence and a second native or heterologous promoter operably linked to the second replication protein coding sequence, preferably wherein the first and second promoters are independently selected from p5, p19, and p40 or an artificial promoter.
14. The rep plasmid according to any one of embodiments 1-13, further comprising a native or heterologous promoter operably linked to the accessory protein coding sequence, preferably wherein the promoter is selected from p5, p19, p40, p81, or an artificial promoter.
15. The rep plasmid according to embodiment 14, wherein the native or heterologous promoter is upstream the accessory protein coding sequence.
16. The rep plasmid according to any one of embodiments 1-15, wherein the rep plasmid does not comprise a capsid protein sequence encoding any functional VP1, VP2, and VP3, or artificial variants thereof.
17. The rep plasmid according to any one of embodiments 1-16, wherein the rep plasmid comprises a capsid protein sequence encoding a non-functional capsid protein sequence.
18. The rep plasmid according to any one of embodiments 1-16, wherein the rep plasmid comprises a capsid coding sequence comprising a silenced start codon.
19. The rep plasmid according to any one of embodiments 1-18, wherein the accessory protein coding sequence is located downstream of the replication protein coding sequence.
20. The rep plasmid according to any one of embodiments 1-19, wherein the rep plasmid comprises a 5'UTR upstream of the accessory protein coding sequence, preferably derived from the sequence located between the native p40 promoter and the native accessory protein coding sequence.
21. The rep plasmid according to any one of embodiments 1-20, wherein the rep plasmid comprises a 3'UTR downstream of the accessory protein coding sequence, preferably derived from the sequence located between the native capsid protein coding sequence and the 3'ITR.
22. The rep plasmid according to any one of embodiments 1-21, wherein the 5' UTR comprises a sequence set forth in SEQ ID NOs: 13 or 14 or a sequence having at least 60%, at least 70%, at least 80% identity, or at least 90% identity to said SEQ ID NOs.
23. The rep plasmid according to any one of embodiments 1-22, wherein the 3' UTR comprises a sequence set forth in SEQ ID NOs: 15, 16, or 17 or a sequence having at least 60%, at least 70%, at least 80%, or at least 90% identity to said SEQ ID NOs.
24. The rep plasmid according to any one of embodiments 1-23, wherein the VP3 start codon in the accessory protein coding sequence is silenced.
25. The rep plasmid according to any one of embodiments 1-24, wherein the rep plasmid comprises one or more polyadenylation signal sequences.
26. The rep plasmid according to any one of embodiments 4-25, wherein the rep78 protein comprises or consists of the SEQ ID NO: 1 or a sequence having at least 60%, at least 70%, at least 80%, or at least 90% identity to said SEQ ID NO.
27. The rep plasmid according to any one of embodiments 4-26, wherein the rep68 protein comprises or consists of the SEQ ID NO: 2 or a sequence having at least 60%, at least 70%, at least 80%, or at least 90% identity to said SEQ ID NO.
28. The rep plasmid according to any one of embodiments 4-27, wherein the rep52 protein comprises or consists of the SEQ ID NO: 3 or a sequence having at least 60%, at least 70%, at least 80%, or at least 90% identity to said SEQ ID NO.
29. The rep plasmid according to any one of embodiments 4-28, wherein the rep40 protein comprises or consists of the SEQ ID NO: 4 or a sequence having at least 60%, at least 70%, at least 80%, or at least 90% identity to said SEQ ID NO.
30. The rep plasmid according to any one of embodiments 7-29, wherein the MAAP protein comprises or consists of the SEQ ID NO: 9 or a sequence having at least 60%, at least 70%, at least 80%, or at least 90% identity to said SEQ ID NO.
31. The rep plasmid according to any one of embodiments 8-30, wherein the AAP protein comprises or consists of the SEQ ID NO: 8 or a sequence having at least 60%, at least 70%, at least 80%, or at least 90% identity to said SEQ ID NO.
32. The rep plasmid according to any one of embodiments 9-31, wherein the X protein comprises or consists of the SEQ ID NO: 10 or a sequence having at least 60%, at least 70%, at least 80%, or at least 90% identity to said SEQ ID NO.
33. The rep plasmid according to any one of embodiments 1-32, wherein the accessory protein coding sequence is from an AVV serotype selected from the group consisting of AAV-1, AAV-2, AAV-3, AAV-4, AAV-5, AAV-6, AAV-7 and AAV-8, preferably selected from AAV2.
34. A plasmid system for producing an adeno-associated virus particle comprising:
   (i) a cap plasmid comprises at least one adeno-associated virus capsid protein coding sequence encoding at least one functional capsid protein, and
   (ii) the rep plasmid of any of embodiments 1 to 33.
35. The plasmid system according to embodiment 34, wherein the cap plasmid comprises a transgene.
36. The plasmid system according to embodiment 34, further comprising a transgene plasmid.
37. The plasmid system according to any one of embodiments 34-36, wherein the system further comprises a helper plasmid, wherein the helper plasmid preferably comprises one or more coding sequences encoding for Ela, Elb, E2A, L7 22k, L433k, E4orf6, E4orf7 fusion, or virus-associated RNAs or any combination thereof.
38. The plasmid system according to embodiment 37, wherein the helper plasmid preferably comprises one or more coding sequences encoding for E2A, E4orf6, E4orf7 fusion, or virus-associated RNAs or any combination thereof.
39. The plasmid system according to embodiment 37 or 38, wherein the helper plasmid comprises one or more virus-associated RNAs.
40. The plasmid system according to any one of embodiments 34-39, wherein the rep plasmid or cap plasmid comprises one or more coding sequences encoding for Ela, Elb, E2A, L7 22k, L433k, E4orf6, E4orf7 fusion, or virus-associated RNAs or any combination thereof.
41. The plasmid system according to cany one of embodiments 34-40, wherein the transgene is a reporter gene, preferably the reporter gene can be detected by antibody-based assays, more preferably the reporter gene is a fluorescent molecule, or the reporter gene is a beta-galactosidase, luciferase, or glutathione S-transferase.
42. The plasmid system according to cany one of embodiments 34-41, wherein the transgene plasmid comprises a promoter, a transgene, a polyadenylation signal sequence, 5' and 3' inverted terminal repeats,
   wherein the transgene plasmid is selected from one of the following:
   (i) conventional single-stranded genome recombinant adeno-associated virus, or
   (ii) self-complementary genome recombinant adeno-associated virus.
43. The plasmid system according to embodiment 42, wherein at least one inverted terminal repeats, preferably the 5' inverted terminal repeats, has a deletion compared to the native inverted terminal repeats.
44. The plasmid system according to any one of embodiments 34-43, wherein the capsid protein coding sequence encodes one or more capsid proteins selected from VP1, VP2, or VP3 or an artificial variant thereof.
45. The plasmid system according to embodiment 44, wherein the VP1 protein comprises or consists of the SEQ ID NO: 5 or a sequence having at least 60%, at least 70%, at least 80%, or at least 90% identity to said SEQ ID NO.
46. The plasmid system according to embodiment 44 or 45, wherein the VP2 protein comprises or consists of the SEQ ID NO: 6 or a sequence having at least 60%, at least 70%, at least 80%, or at least 90% identity to said SEQ ID NO.
47. The plasmid system according to any one of embodiments 44-46, wherein the VP3 protein comprises or consists of the SEQ ID NO: 7 or a sequence having at least 60%, at least 70%, at least 80%, or at least 90% identity to said SEQ ID NO.
48. A stable or transient cell expression system comprising the plasmid system according to any one of embodiments 34-47 and a cell line.
49. A cell comprising the rep plasmid according to any of embodiments 1-33 or the plasmid system according to any one of embodiments 34-47.
50. A kit comprising a stable or transient cell expression system according to embodiment 48, or a cell according to embodiment 49, and a cell culture medium.
51. The kit according to embodiment 50, further comprising media feeds, media additives or transfection reagents or any combination thereof.
52. The kit according to embodiment 50 or 51, further comprising a manual.
53. A method of producing recombinant adeno-associated viral vectors comprising:
   (i) transfecting cells with the rep plasmid according to any of embodiments 1-33, or the plasmid system according to any one of embodiments 34-47;
   (ii) culturing the transfected cells to produce said adeno-associated viral vectors; and
   (iii) isolating said recombinant adeno-associated viral vectors.
54. The method according to embodiment 53, wherein the cells are HEK293 cells, HELA cells, CHO cells, insect cells, or derivatives thereof.

### Definitions

The terms indicated for explanation of the invention have the following meaning, unless otherwise indicated in the description or the claims. Additional definitions are set forth throughout the detailed description.

The "3'UTR sequence" is a 3' untranslated region known to regulate mRNA-based processes, such as mRNA localization, mRNA stability, and translation. In addition, 3' UTRs can establish 3' UTR-mediated protein-protein interactions (PPIs), and thus can transmit genetic information encoded in 3' UTRs to proteins. This function has been shown to regulate diverse protein features, including protein complex formation or posttranslational modifications, but is also expected to alter protein conformations.

The "5'UTR sequence" is a 5'-untranslated region which lies within the noncoding genome upstream of a coding sequence and plays an important role in regulating gene expression. Within 5'-UTR sequences may be numerous cis-regulatory elements present that can interact with the transcriptional machinery to regulate mRNA abundance. The 5'-untranslated region may contain various RNA-based regulatory elements including the secondary structures, RNA-binding protein motifs, upstream open-reading frames (uORFs), internal ribosome entry sites, terminal oligo pyrimidine (TOP) tracts, and G-quadruplexes.

These elements can alter the efficiency of mRNA translation; some can also affect mRNA transcript levels via changes in stability or degradation.

Terms "a" and "an" and "the" and similar reference used in the context of describing the invention (especially in the context of the claims) are to be construed to cover both the singular and the plural, unless otherwise indicated herein or clearly contradicted by context.

The terms "about" or "approximately" as used herein denotes a range of ±10% of a reference value. For examples, "about 10" defines a range of 9 to 11. In general, those skilled in the art, familiar with the context, will appreciate the relevant degree of variance encompassed by "about" or "approximately" in that context.

As used herein, the term "adeno-associated virus" (AAV), includes but is not limited to, AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

A "cap plasmid" is a plasmid comprising a capsid protein coding sequence encoding a functional cap protein. The terms "cap plasmid" and "capsid plasmid" can be used interchangeably herein. As used herein, the cap plasmid preferably is not capable of encoding a functional rep protein. It is understood by a skilled person that this can be achieved in various ways. For example, the cap plasmid may comprise a coding sequence encoding a non-functional rep protein or may comprise a non-functional replication protein coding sequence which is not capable of being transcribed e.g., due to a silenced start codon. In some embodiments, the cap plasmid does not comprise any replication protein coding sequence.

Unless expressly specified otherwise, the term "comprising" is used in the context of the present disclosure to indicate that further members may optionally be present in addition to the members of the list introduced by "comprising". It is, however, contemplated as specific embodiments of the present invention that each time the term "comprising" is used, this shall also encompass the possibility of no further members being present, i.e., for the purpose of such specific embodiments "comprising" can be understood as having the meaning of "consisting of'.

As used herein "derived from" in the context of a nucleic acid or amino acid sequence means that the sequence is identical to a sequence from which it is derived or has a specified percentage of amino acid residues or nucleotides that are the same as the sequence from which it is derived. The specified percentage can be about at least 60%, preferably at least 65%, at least 70%, at least 75%, at least 80%, at least 85%, at least 90%, at least 91%, at least 92%, at least 93%, at least 94%, at least 95%, at least 96%, at least 97%, at least 98%, at least 99% over a specified sequence, when compared and aligned for maximum correspondence over a comparison window or designated region as measured using a BLAST or BLAST 2.0 sequence comparison algorithms with default parameters, or by manual alignment and visual inspection. For example, the 5'UTR can be derived from a specific sequence of e.g., AAV2, preferably J01901.1 AAV2, e.g., the 5'UTR sequence can be identical to the 5'UTR sequence present between the p40 promoter and the e.g., MAAP coding sequence, comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 5'UTR sequence comprised in the AAV2 genome, preferably J01901.1 AAV2 genome. The 3'UTR can be derived from a specific sequence of e.g., AAV2, preferably J01901.1 AAV2, e.g., the 3' UTR sequence can be identical to the 3' UTR sequence present between the capsid gene and the 3'ITR comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 3'UTR sequence comprised in the AAV2 genome, preferably J01901.1 AAV2 genome. In the context of this disclosure, it is understood that "J01901.1" refers to the GenBank accession code "J01901.1" of the wild-type AAV2 complete genome set forth in SEQ ID NO: 21.

Herein, the term "DNA" relates to a nucleic acid molecule which is entirely or at least substantially composed of deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

A "feed" or "supplement" as used herein refers to a composition when added to cells in standard culture may be beneficial for its maintenance, or expansion, or growth, or viability, or affects its cell performance, or increases culture longevity or maintaining cells in a pseudo-stationary phase wherein product expression continues, or results in an increase in final product titer. A feed or supplement may be used interchangeably in this disclosure and refers to solid and liquid formats (including agglomerated formats) of media components comprising one or more amino acids, sugars, vitamins, buffers, sometimes, peptides, hydrolysates, fractions, growth factors, hormones, etc. required to rebalance or replenish or to modulate the growth or performance of a cell in culture, or a cell culture system. A feed or supplement may be distinguished from a cell culture medium in that it is added to a cell culture medium that can culture a cell. As would be understood by one of skill in the art, sometimes a feed/supplement may comprise mainly those amino acids, sugars, vitamins, buffers, etc. required to rebalance or replenish or modulate the growth or performance of a cell in culture, or a cell culture system. A feed or supplement may or may not be concentrated or may be partially concentrated for certain components only.

As used herein, the term "gene" refers to the segment of a DNA molecule that codes for a polypeptide chain (e.g., the coding region). In some embodiments, a gene is positioned by regions immediately preceding, following, and/or intervening the coding region that are involved in producing the polypeptide chain (e.g., regulatory elements such as a promoter, enhancer, polyadenylation sequence, 5'-untranslated region (5'UTR), 3'-untranslated region (3'UTR), or intron).

As used herein, the term "encode" or "encoding" refers to sequence information of a first molecule that guides production of a second molecule having a defined sequence of nucleotides (e.g., mRNA) or a defined sequence of amino acids. For example, a DNA molecule can encode an RNA molecule (e.g., by a transcription process that includes a DNA-dependent RNA polymerase enzyme). A coding sequence encoding a protein is a sequence that guides production of said protein. Thus, a coding sequence, a gene, a cDNA, or a single-stranded RNA (e.g., an mRNA) encodes a polypeptide if transcription and translation of mRNA corresponding to that gene produces the polypeptide in a cell or other biological system. In some embodiments, a coding sequence encoding a target polypeptide refers to a coding strand, the nucleotide sequence of which can be identical to the mRNA sequence of such a target polypeptide. In some embodiments, a coding sequence encoding a target polypeptide refers to a non-coding strand of such a target polypeptide agent, which may be used as a template for transcription of a gene or cDNA. As is understood in the art, the phrase "coding sequence encoding a peptide or protein" means that the plasmid containing the coding sequence, if present in the appropriate environment, for example within a cell and/or in a cell-free translation system, can direct the assembly of amino acids to produce the peptide or protein via a process of translation.

A functional rep protein in context of the invention is a replication (rep) protein involved in AAV replication and/or AAV assembly. The terms "replication protein" and "rep protein" are used synonymously in the context of the present disclosure. It is within the abilities of the skilled person to determine whether a given rep protein is functional. The functionality of rep78, rep68, rep52, and/or rep40 polypeptides or artificial variants thereof may be determined by the skilled person using PCR-based techniques, e.g., by droplet digital PCR (ddPCR) or real-time PCR (qPCR), or by Western Blot. In some embodiments, the functional rep protein is involved in AAV replication and viral assembly. In some embodiments, the functional rep protein is involved in AAV replication. In some embodiments, the functional rep protein is involved in AAV virion assembly. In some embodiments, the functional rep protein binds to DNA. In some embodiments, the functional rep protein does not bind to DNA.

A functional cap protein in context of this disclosure is a cap protein capable of forming a capsid. The terms "capsid protein" and "cap protein" are used synonymously in the context of the present disclosure. A functional cap protein can be any of VP1, VP2, VP3 or artificial variants thereof capable of forming a capsid. It is within the abilities of the skilled person to determine whether a given cap protein is functional. For example, a test two-plasmid system can comprise a plasmid that comprises the cap gene which encodes the protein(s) whose functionality is to be determined, and otherwise the test two-plasmid system used will be identical to a reference two-plasmid system. The reference two-plasmid system preferably comprises the wild-type cap coding sequence preferably encoding one or more of VP1, VP2, and VP3 having the amino acid sequences shown in SEQ ID NO: 5-7. The rep plasmid does not comprise a cap gene encoding a functional cap protein or is not capable of encoding a functional cap protein if, e.g., the rep plasmid does not comprise any genetic material corresponding to a cap gene, or if any cap coding sequence present in the plasmid encodes cap protein(s) which support AAV production at a level below 10%, preferably below 5%, more preferably below 1% of the level supported by the wild type cap gene product (e.g., VP1, VP2, and VP3 shown in SEQ ID NO: 5-7), i.e., the yield of produced rAAV is less than 10%, preferably less than 5%, more preferably less than 1% of the yield of rAAV produced using the reference two-plasmid system. The skilled person knows how to determine rAAV particle formation and thus formation of functional capsids, e.g., by Enzyme-linked Immunosorbent Assay (ELISA), differential scanning fluorimetry (DSF), chromatographic methods, or spectroscopic methods (e.g., bio-layer interferometry and/or surface plasmon resonance spectroscopy). For example, methods to verify functional capsid assembly are described in Green and Kelvin, "Analytical methods to characterize recombinant adeno-associated virus vectors and the benefit of standardization and reference materials ", Curr Opin Biotechnol. 2021 Oct; 71: 65-76, doi: 10.1016/j.copbio.2021.06.025. In general, a functional cap gene is greater than 250 nucleotides in length. Accordingly, the plasmid, which does not comprise a cap gene encoding a functional set of cap proteins, may not comprise a contiguous stretch of exclusively cap gene sequence of more than 250 nucleotides, more than 100 nucleotides, or more than 60 nucleotides. Optionally, the rep plasmid does not comprise a contiguous stretch of exclusively cap gene sequence of more than 60 nucleotides. Optionally the rep plasmid does not comprise a cap gene encoding a functional VP1, VP2, and/or VP3 protein. Optionally, the rep plasmid comprises a portion of cap gene sequence, and the portion of cap gene sequence does not encode any cap protein. The rep plasmid can comprise a (portion of) cap gene sequence, so long as it does not encode a functional cap protein.

The term "helper" is not intended to be limiting. Accordingly, a "helper plasmid" is any plasmid that comprises at least one helper virus gene. In some embodiments, a helper virus can be selected from Ela, Elb, E2A, L7 22k, L433k, E4orf6, E4orf7 fusion, or virus-associated RNAs or any combination thereof

The terms "identical" or percent (%) "identity," in the context of two or more nucleic acids or peptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection.

Inverted terminal repeats (ITRs) are guanine-cytosine-rich structures involved in the replication and encapsidation of the AAV genome, along with its integration in and excision from the host genome. ITRs are natural AAV-derived DNA sequences conserved in recombinant AAV (rAAV), as they allow its replication, encapsidation, and long-term maintenance and expression in target cells. ITRs can be incomplete, truncated, and/or modified. An ITR can be a truncated, meaning one or more nucleotides of the natural sequence can be deleted. An ITR can be modified, meaning an ITR can comprise one or more nucleotides of non-ITR sequences. The non-ITR sequence can be inserted within the ITR sequence or the non-ITR sequence can be added at one or both ends of the ITR sequence. The modified ITR sequence can comprise substitution wherein one or more nucleotides are exchanged. It has been shown that rAAV genomes can be replicated, even with incomplete, truncated, or modified ITR sequences. It is understood that the present invention is not limited to a specific ITR sequence and any ITR sequence now known or later discovered can be used.

As used herein, a nucleic acid sequence (e.g., coding sequence) and regulatory sequences are said to be "operatively linked" when they are covalently linked in such a way as to place the expression or transcription of the nucleic acid sequence under the influence or control of the regulatory sequences. If it is desired that the nucleic acid sequences be translated into a functional protein, two DNA sequences are said to be operatively linked if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein.

A "polyadenylation signal sequence" (PAS or poly(A)) is a sequence known to those skilled in the art. The polyadenylation signal sequence typically comprises a conserved hexamer motif required for the polyadenylation of an mRNA, a U rich and/or GU rich sequence downstream of the hexamer motif, and a dinucleotide sequence that precedes the cleavage site for polyadenlation and is located between the U rich and/or GU rich sequence and the hexamer motif. The sequence can be recognized by cleavage and polyadenylation specificity factor (CPSF) within an RNA cleavage complex. The hexamer motif varies between eukaryotes but can be AATAAA or modifications thereof. CPSF is the central component of the 3' processing machinery for polyadenylated mRNAs and recognizes the PAS, thereby providing sequence specificity in both pre-mRNA cleavage and polyadenylation, and catalyzes pre-mRNA cleavage.

A "poly(A)-tail" (or "poly(A)-sequence") is an adenine nucleotide chain typically added to a mRNA molecule during RNA processing to increase the stability of the molecule and enable translation. This process, called polyadenylation, usually adds 100 to 250 adenines.

The terms "nucleic acid sequence", "nucleotide sequence", "polynucleotide" and "nucleic acid" can be used interchangeably herein to refer to one or more nucleotides, preferably deoxyribonucleic acid (DNA). These terms comprise DNA, ribonucleic acid (RNA), combinations thereof, and modified forms thereof. The term comprises genomic DNA, cDNA, mRNA, recombinantly produced and chemically synthesized molecules. In some preferred embodiments, a polynucleotide is DNA. In some embodiments, a polynucleotide is a mixture of DNA and RNA. A polynucleotide may be present as a single-stranded or double-stranded and linear or covalently circularly closed molecule. A polynucleotide can be isolated. The term "isolated polynucleotide " means, according to the present disclosure, that the polynucleotide (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "plasmid" refers to an extrachromosomal, mostly circular DNA capable of being expressed in a given cell. Plasmids can also be engineered by standard molecular biology techniques (Sambrook et al., Laboratory Manual, Cold Spring Harbor Laboratory Press, Cold Spring Harbor (1989), N.Y.).

The terms "polypeptide," "peptide," and "protein" are used interchangeably herein to refer to polymers of amino acids.

The term "recombinant" when used in the context of a polynucleotide is a polynucleotide having nucleotide sequences that are not naturally joined together and can be made by artificially combining two otherwise separated segments of sequence. This artificial combination is often accomplished by chemical synthesis or, more commonly, by the artificial manipulation of isolated segments of nucleic acids, for example, by genetic engineering techniques. Recombinant polynucleotides include vectors comprising an amplified or assembled polynucleotide, which can be used to transform or transfect a suitable host cell. A host cell that comprises the recombinant polynucleotide is a "recombinant host cell." The polynucleotide is then expressed in the recombinant host cell to produce a "recombinant polypeptide." A recombinant polynucleotide can also comprise a non-coding function.

A "rep plasmid" is a plasmid comprising a replication protein coding sequence encoding a functional rep protein. The terms "rep plasmid" and "replication plasmid" can be used interchangeably herein.

A "rep cap plasmid" or "rep-cap plasmid" is a plasmid comprising a replication protein coding sequence encoding a functional rep protein and a capsid protein coding sequence encoding a functional cap protein. The terms "rep cap plasmid" and "replication capsid plasmid" can be used interchangeably herein.

"Recombinant AAV" (rAAV) and "AAV" are used interchangeably throughout the application.

As used herein, a "transgene" is a nucleic acid that is introduced into the cell, including but not limited to genes or nucleic acid having sequences which are not normally present in AAV, genes which are present but not normally transcribed and translated ("expressed") in an AAV genome, or any other gene or nucleic acid which one desires to position between the ITR sequences. A transgene may include one or more transcriptional regulatory sequences and any other nucleic acid, such as introns, that may be necessary for optimal expression of a selected nucleic acid. A transgene can be as few as a couple of nucleotides long e.g., less than 50 or less than 30 nucleotides long, but can preferably be at least about 50, 100, 150, 200, 250, 300, 350, 400, 500, 600, 700, 800, 900, 1.000, 1.100, 1.200, 1.300, 1.400, 1.500, 1.600, 1.700, 1.800, 1.900, 2.000, 2.100, 2.200, 2.300, 2.400, 2.500, 2.600, 2.700, 2.800, 2.900, 3.000, 3.100, 3.00, 3.300, 3.400, 3.500, 3.600, 3.700, 3.800, 3.900, 4.000, 4.100, 4.200, 4.300, 4.400, 4.500, or 4.600 nucleotides (nt) long.. A transgene can comprise coding or non-coding sequences.

As used herein, the term "variant" refers to a molecule, such as a gene or protein, that shares one or more particular structural features, elements, components, or moieties with a reference molecule. A variant therefore is a molecule, such as a gene or protein, that shares one or more particular structural features, elements, components, or moieties with a reference molecule. A variant can be a gene that shares one or more particular structural features, elements, components, or moieties with a reference gene. A variant can be a protein that shares one or more particular structural features, elements, components, or moieties with a reference protein. Typically, a "variant" has significant structural similarity with the reference molecule, for example sharing a core or consensus structure, but also differs in certain discrete ways. In some embodiments, a variant can be an artificial variant that has been altered by human intervention. In some embodiments, an artificial variant is a molecule that can be generated from the reference molecule, e.g., by chemical manipulation of the reference molecule. In some embodiments, an artificial variant is a molecule that can be generated from the reference molecule, e.g., by genetic engineering techniques. In some embodiments, a variant is a molecule that can be generated through performance of a synthetic process substantially similar to (e.g., sharing a plurality of steps with) one that generates the reference molecule. In some embodiments, a variant is or can be generated through performance of a synthetic process different from that used to generate the reference molecule.

As used herein, the terms "virus vector," "viral vector," and "gene delivery vector" refer to a virus particle that functions as a nucleic acid delivery vehicle, and which comprises a nucleic acid molecule packaged within a viral capsid. Exemplary virus vectors include adeno-associated virus (AAV) vectors.

As used herein, the term "transduction efficiency" refers to the ability of a viral vector or viral particle, preferably an rAAV particle, to transduce a target cell. As used herein, the term "potency" refers to the ability of a viral vector or viral particle, preferably an rAAV particle, to transduce a target cell and express a transgene in the target cell. Potency can be represented in transducing units (e.g., TU per volume). Transducing unit as used herein refers to the number of functional viral vectors or viral particles, preferably rAAV particles, in a solution that are capable of transducing a target cell and expressing a transgene in the target cell. The skilled person knows how to determine the potency of a viral vector or a viral particle. For example, potency can be determined according to the methods described in Green and Kelvin, "Analytical methods to characterize recombinant adeno-associated virus vectors and the benefit of standardization and reference materials ", Curr Opin Biotechnol. 2021 Oct; 71: 65-76, doi: 10.1016/j.copbio.2021.06.025.

The terms "wild type" and "native" as used herein are synonymous and refer to genes present in the genome of a strain/serotype of AAV or adenovirus, or to proteins encoded by genes present in the genome of a strain/serotype of AAV or adenovirus. Preferably, the wild type strain/serotype of AAV or adenovirus can refer to the AAV2 genome shown in the GenBank accession code J01901.1.

Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

References to "one embodiment," "an embodiment," "example embodiment," "some embodiments," "certain embodiments," "various embodiments," etc., indicate that the embodiment(s) of the disclosed technology so described may include a particular feature, structure, or characteristic, but not every embodiment necessarily includes the particular feature, structure, or characteristic.

All patents, patent applications, and other publications cited in this application are incorporated by reference in the entirety for all purposes.

### The rep plasmid of the invention

The present invention provides a rep plasmid comprising:
(i) at least one adeno-associated virus replication protein coding sequence encoding at least one functional rep protein; and
(ii) at least one accessory protein coding sequence;
wherein the rep plasmid is not capable of encoding a functional cap protein.

It has been surprisingly found that adding an accessory protein coding sequence encoding at least one functional accessory protein improves the transducing titer of the rAAVs produced using a rep/cap split plasmid system of the invention. As demonstrated in the examples (see example 2), the accessory protein coding sequence leads to an improved transducing titer and thus, to rAAVs with improved potency. For this reason, the rep plasmid disclosed herein is particularly suitable for rAAV production using the flexible rep/cap split plasmid system, usually resulting in a lower rAAV transducing titer compared to a system comprising rep and cap genes on a single plasmid encoding functional rep and cap proteins.

The accessory protein can be selected from AAP (Assembly-Activating Protein), MAAP (Membrane-Associated Accessory Protein), and X or variants thereof or any combination thereof. The CDS of the accessory proteins are overlapping the capsid gene in the wild-type AAV. AAP is suggested to be important for assembly of the capsid and maintaining capsid protein stability (Grosse, Stefanie et al. "Relevance of Assembly-Activating Protein for Adeno-associated Virus Vector Production and Capsid Protein Stability in Mammalian and Insect Cells." Journal of virology vol. 91,20 e01 198-17. 27 Sep. 2017, doi: 10.1128/JVI.01198-17). MAAP is commonly translated from a non-canonical start codon, CTG, and is suggested to be associated with the cell membrane. MAAP is contemplated to limit the production of other AAV strains through competitive exclusion (Karlin, D.G. Sequence Properties of the MAAP Protein and of the VP1 Capsid Protein of Adeno-Associated Viruses. Preprints 2020, 2020020234). The function of X is unknown, although X is suggested to be involved in DNA replication and DNA binding (Cao M, You H, Hermonat PL (2014) The X Gene of Adeno-Associated Virus 2 (AAV2) Is Involved in Viral DNA Replication. PLoS ONE 9(8): e104596).

In preferred embodiments, the accessory protein can be selected from MAAP, and X or variants thereof or any combination thereof. In most preferred embodiments, the accessory protein is at least MAAP and/or a variant thereof.

The wild-type cap gene comprises 6 open reading frames: VP1, VP2, VP3, AAP, MAAP, and X. Thus, in the prior art rep/cap split plasmid systems in which the rep plasmid does not comprise the cap gene, the accessory genes are not encoded on the rep plasmid, but the accessory protein expression is provided in trans to the rep plasmid, i.e., provided by a separate plasmid. Without wishing to be bound by theory, providing at least one accessory protein expression in cis to the rep plasmid is contemplated to improve the transducing titer (and thus, also potency) of the produced rAAV.

In some embodiments, the accessory protein coding sequence transcribes a functional accessory protein mRNA. In some embodiments, the accessory protein coding sequence encodes an accessory protein. In some embodiments, the accessory protein coding sequence encodes one or more of AAP, MAAP, and X, or artificial variants thereof, preferably MAAP, and X, or artificial variants thereof, more preferably MAAP or artificial variants thereof.

In some preferred embodiments, the accessory protein coding sequence encodes MAAP or an artificial variant thereof. In some embodiments, the accessory protein coding sequence encodes X or an artificial variant thereof. In some embodiments, the accessory protein coding sequence encodes AAP or an artificial variant thereof. The rep plasmid disclosed herein can comprise any combination of accessory protein coding sequences. For example, the rep plasmid can comprise a first accessory protein coding sequence encoding MAAP or an artificial variant thereof, and a second accessory protein coding sequence encoding X or an artificial variant thereof. The rep plasmid can comprise a first accessory protein coding sequence encoding AAP or an artificial variant thereof, and a second accessory protein coding sequence encoding X or an artificial variant thereof. The rep plasmid can comprise a first accessory protein coding sequence encoding MAAP or an artificial variant thereof, and a second accessory protein coding sequence encoding AAP or an artificial variant thereof. The rep plasmid can comprise a first accessory protein coding sequence encoding MAAP or an artificial variant thereof, a second accessory protein coding sequence encoding X or an artificial variant thereof, and a third accessory protein coding sequence encoding AAP. Most preferably, the rep plasmid comprises at least one accessory protein coding sequence encoding at least MAAP or an artificial variant thereof.

In some embodiments, the accessory protein coding sequence encodes one or more of AAP, MAAP, and X, or artificial variants thereof, wherein AAP, MAAP, and X comprises or consists of one or more of the sequences shown in SEQ ID NO: 8-10 or a sequence having at least 60%, at least 70%, at least 80%, or at least 90% identity to said SEQ ID NOs. In some preferred embodiments, the accessory protein coding sequence encodes one or more of MAAP, and X, or artificial variants thereof, wherein MAAP, and X comprises or consists of one or more of the sequences shown in SEQ ID NO: 9 and 10 or a sequence having at least 60%, at least 70%, at least 80%, or at least 90% identity to said SEQ ID NOs.

In some embodiments, the MAAP protein comprises or consists of the SEQ ID NO: 9 or a sequence having at least 60%, at least 70%, at least 80%, or at least 90% identity to said SEQ ID NO.

In some embodiments, the AAP protein comprises or consists of the SEQ ID NO: 8 or a sequence having at least 60%, at least 70%, at least 80%, or at least 90% identity to said SEQ ID NO.

In some embodiments, the X protein comprises or consists of the SEQ ID NO: 10 or a sequence having at least 60%, at least 70%, at least 80%, or at least 90% identity to said SEQ ID NO.

The rep plasmid can comprise at least one replication protein coding sequence encoding at least two functional rep proteins, at least three functional rep proteins, or at least four functional rep proteins. The rep plasmid can comprise at least two replication protein coding sequences, each encoding at least one functional rep protein, preferably wherein the rep plasmid comprises two replication protein coding sequences, each encoding one functional rep protein. In some embodiments, the replication protein coding sequence encodes one or more of rep68, rep78, rep52, and rep40 or artificial variants thereof. For example, the rep plasmid comprises a first replication protein coding sequence encoding the functional rep protein selected from rep68 and rep78 or artificial variants thereof, and a second replication protein coding sequence encoding the functional rep protein selected from rep52 and rep40 or artificial variants thereof.

In some embodiments, the replication protein coding sequence encodes rep68 or an artificial variant thereof. In some embodiments, the replication protein coding sequence encodes rep78 or an artificial variant thereof. In some embodiments, the replication protein coding sequence encodes rep52 or an artificial variant thereof. In some embodiments, the replication protein coding sequence encodes rep40 or an artificial variant thereof.

In some embodiments, the rep protein comprises or consists of any of the sequences shown in SEQ ID NO: 1-4 or a sequence having at least 60%, at least 70%, at least 80%, or at least 90% identity to said SEQ ID NOs. In some embodiments, the rep78 protein comprises or consists of the SEQ ID NO: 1 or a sequence having at least 60%, at least 70%, at least 80%, or at least 90% identity to said SEQ ID NO. In some embodiments, the rep68 protein comprises or consists of the SEQ ID NO: 2 or a sequence having at least 60%, at least 70%, at least 80%, or at least 90% identity to said SEQ ID NO. In some embodiments, the rep52 protein comprises or consists of the SEQ ID NO: 3 or a sequence having at least 60%, at least 70%, at least 80%, or at least 90% identity to said SEQ ID NO. In some embodiments, the rep40 protein comprises or consists of the SEQ ID NO: 4 or a sequence having at least 60%, at least 70%, at least 80%, or at least 90% identity to said SEQ ID NO.

In some embodiments, the rep plasmid further comprises regulatory sequences including promoter, binding sites, and/or non-coding RNA. These sequences can be independently derived from AAV, including different AAV serotypes.

In some embodiments, the rep plasmid can comprise one or more rep-binding elements (RBE). A RBE can function as binding site for rep68 and/or rep78. In some embodiments, the one or more RBEs function as binding site for rep68 or rep78. In some embodiments, the one or more RBEs function as binding site for rep68 and rep78. In some embodiments, the one or more RBEs function as binding site for rep68. In some embodiments, the one or more RBEs function as binding site for rep78. In some embodiments, the RBE is derived from a non-ITR sequence, such as an AAV promoter sequence. In some embodiments, the RBE is derived from a p5 promoter. In some embodiments, the RBE is comprised in a heterologous element, meaning in a sequence which is not native to the AAV genome. In some preferred embodiments, the RBE is derived from a p5 promoter but is not comprised in a functional p5 promoter. In some embodiments, the RBE is derived from an ITR sequence but is not comprised in a functional ITR sequence. The RBE can be preferably located downstream the accessory protein coding sequence and upstream a polyadenylation sequence, wherein the polyadenylation sequence preferably is a heterologous polyadenylation sequence, meaning a polyadenylation sequence which is not comprised in the wild type AAV genome.

The RBE may preferably comprise or consist of the SEQ ID NO: 12 or a sequence having at least 60%, at least 70%, at least 80%, or at least 90% identity to said SEQ ID NO. The RBE may comprise or consist of the SEQ ID NO: 18 or a sequence having at least 60%, at least 70%, at least 80%, or at least 90% identity to said SEQ ID NO.

The rep proteins are generally translated from transcripts whose transcription is regulated by the p5 and p19 promoter. In some embodiments, the rep plasmid comprises a native or heterologous promoter operably linked to the replication protein coding sequence, preferably wherein the promoter is selected from p5, p19, and p40 or an artificial promoter.

The promoter can be derived from a different serotype than the replication protein coding sequence. The promoter can be derived from a different serotype than the accessory protein coding sequence. In some embodiments, the rep plasmid can comprise a non-natural promoter, meaning a promoter which is not derived from AAV. In some embodiments, the rep plasmid comprises a mouse mammary tumor virus (MMTV) promoter. In some embodiments, a MMTV promoter is operatively linked to the replication protein coding sequence. For example, the rep plasmid may comprise a MMTV promoter operably linked to the replication protein coding sequence.

In some embodiments, the rep plasmid comprises a first native or heterologous promoter operably linked to the first replication protein coding sequence and a second native or heterologous promoter operably linked to the second replication protein coding sequence, preferably wherein the first and second promoters are independently selected from p5, p19, and p40 or an artificial promoter. The rep plasmid can e.g., comprise a first replication protein coding sequence operably linked to MMTV and encoding a functional rep protein selected from rep68 and rep78 or artificial variants thereof, and a second replication protein coding sequence operatively linked to p19 and encoding a functional rep protein selected from rep52 and rep40 or artificial variants thereof.

In some embodiments, the rep plasmid comprises a native or heterologous promoter operably linked to the accessory protein coding sequence, preferably wherein the promoter is selected from p5, p19, p40, p81 or an artificial promoter. Preferably, the native or heterologous promoter is located upstream of the accessory protein coding sequence. For example, the native or heterologous promoter and a 5'UTR sequence can be located upstream the accessory protein coding sequence. By convention, upstream and downstream relate to the 5' to 3' direction respectively in which RNA transcription can take place. "Upstream" is toward the 5' end of the RNA molecule and downstream is toward the 3' end. When considering double-stranded DNA, upstream is toward the 5' end of the coding strand for the gene in question and downstream is toward the 3' end of the coding strand for the gene in question.

In some embodiments, the rep plasmid comprises a functional p40 promoter. A functional p40 promoter is one that is capable to drive expression. Whether or not a given p40 promoter is functional may be determined by testing its ability to drive expression of a protein (using an expression assay). For example, a skilled person may prepare a reference plasmid comprising the p40 promoter upstream of a reporter protein such as GFP. A p40 promoter will be considered to be functional if its drives expression at a level at least 30% of the expression driven by the native p40 promoter. Optionally, a functional p40 promoter drives expression at a level at least 40%, at least 50%, at least 60%, at least 70%, or at least 80% of the expression driven by the native p40 promoter. For example, the rep plasmid can comprise a MAAP and/or AAP coding sequence operatively linked to a p40 promoter or an artificial variant thereof.

In some embodiments, the rep plasmid comprises a functional p81 promoter. For example, the rep plasmid can comprise a X coding sequence operatively linked to a p81 promoter or an artificial variant thereof.

The rep plasmid can preferably comprise one of the following structures:
(1) 5' - promoter - accessory protein coding sequence - 3'
(2) 5' - promoter - accessory protein coding sequence - RBE - 3'
(3) 5' - promoter - accessory protein coding sequence - PAS - 3'
(4) 5' - promoter - 5'UTR - accessory protein coding sequence - 3'
(5) 5' - promoter - 5'UTR - accessory protein coding sequence - RBE - 3'
(6) 5' - promoter - 5'UTR - accessory protein coding sequence - PAS - 3'
(7) 5' - promoter - 5'UTR - accessory protein coding sequence - PAS - RBE - PAS - 3'
(8) 5' - p40 - 5'UTR - accessory protein coding sequence - 3'
(9) 5' - p40 - 5'UTR - accessory protein coding sequence - RBE - 3'
(10) 5' - p40 - 5'UTR - accessory protein coding sequence - PAS - 3'
(11) 5' - p40 - 5'UTR - accessory protein coding sequence - PAS - RBE - PAS - 3'
(12) 5' - p40 - 5'UTR - MAAP coding sequence - 3'
(13) 5' - p40 - 5'UTR - MAAP coding sequence - RBE - 3'
(14) 5' - p40 - 5'UTR - MAAP coding sequence - PAS - 3'
(15) 5' - p40 - 5'UTR - MAAP coding sequence - PAS - RBE - PAS - 3'

The rep plasmid can preferably comprise one of the following structures in addition or in alternative to any of the above structures (1)-(15)
(16) 5' - p81 - 5'UTR - accessory protein coding sequence - 3'
(17) 5' - p81 - 5'UTR - accessory protein coding sequence - RBE - 3'
(18) 5' - p81 - 5'UTR - accessory protein coding sequence - PAS - 3'
(19) 5' - p81 - 5'UTR - accessory protein coding sequence - PAS - RBE- PAS - 3'
(20) 5' - p81 - 5'UTR - X coding sequence - 3'
(21) 5' - p81 - 5'UTR - X coding sequence - RBE - 3'
(22) 5' - p81 - 5'UTR - X coding sequence - PAS - 3'
(23) 5' - p81 - 5'UTR - X coding sequence - PAS - RBE - PAS - 3'

The above constructs can comprise a 3' UTR. The PAS and the RBE provided in any of structures (2), (3), (5)-(7), (9)-(11), (13)-(15), (17)-(19), and (21)-(23) can be preferably comprised in a 3' UTR sequence. The RBE is preferably derived from a p5 promoter sequence as shown in SEQ ID NO: 12. The polyadenylation signal sequence (PAS) can either be selected from a native and/or a heterologous polyadenylation signal sequence. In some embodiments, the 3' UTR comprises two polyadenylation signal sequences. In such embodiments, the at least two polyadenylation signal sequences can either be the same polyadenylation signal sequence or can be different polyadenylation signal sequences. For example, the 3' UTR can comprise a first native polyadenylation signal sequence and a second heterologous polyadenylation signal sequence, e.g., a rabbit β-globin poly(A) signal sequences as shown in SEQ ID NO:19. The 5' UTR is preferably derived from the sequence located between the native p40 promoter and the native accessory protein coding sequence.

In some embodiments, the replication protein coding sequence is derived from an AAV serotype selected from the group consisting of AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In some embodiments, the replication protein coding sequence is from an AVV serotype selected from the group consisting of AAV-1, AAV-2, AAV-3, AAV-4, AAV-5, AAV-6, AAV-7, and AAV-8, preferably selected from AAV2.

In some embodiments, the accessory protein coding sequence is derived from an AAV serotype selected from the group consisting of AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In some embodiments, the accessory protein coding sequence is from an AVV serotype selected from the group consisting of AAV-1, AAV-2, AAV-3, AAV-4, AAV-5, AAV-6, AAV-7, and AAV-8, preferably selected from AAV2.

According to the invention, the rep plasmid is not capable of encoding a functional cap protein. It is however understood by a skilled person that this can be achieved in various ways. In some embodiments, the rep plasmid comprises a non-functional coding sequence. A non-functional coding sequence is a coding sequence which is not capable of being transcribed. In some embodiments, the rep plasmid comprises a functional coding sequence which however is not capable of encoding a functional cap protein. For example, the rep plasmid may comprise a coding sequence encoding a non-functional cap protein or may comprise a coding sequence comprising a silenced start codon. A silenced start codon is a start codon which does not lead to gene expression, by e.g., removal of the start codon or mutation of the start codon. In some embodiments, the coding sequence can be transcribed but may not result in a functional mRNA which can be translated. In some embodiments, the coding sequence can be transcribed, and the resulting mRNA can be translated but the resulting cap protein is non-functional, e.g., due to mutations and/or truncations. In some embodiments, the rep plasmid does not comprise a capsid coding sequence. It is understood in the context of this disclosure, that the rep plasmid comprises at least one accessory protein coding sequence which in a wild-type AAV is overlapping with one or more capsid coding sequences. The rep plasmid disclosed herein therefore comprises at least a truncated capsid coding sequence which is however not capable of encoding functional cap protein.

In some embodiments, the rep plasmid is not capable of encoding any functional cap protein (i.e., VP1, VP2, and VP3, and artificial variants thereof). In some embodiments, the rep plasmid comprises a capsid protein sequence encoding a non-functional cap protein. In some embodiments, the rep plasmid comprises a capsid protein sequence encoding a non-functional truncated cap protein. In some embodiments, the rep plasmid comprises a capsid coding sequence comprising a silenced start codon. In some preferred embodiments, the VP2 start codon in the accessory protein coding sequence is silenced. In some preferred embodiments, the VP3 start codon in the accessory protein coding sequence is silenced. In some preferred embodiments, the accessory protein coding sequence is a MAAP coding sequence, wherein the VP2 start codon in the accessory protein coding sequence is silenced. In some embodiments, the accessory protein coding sequence is an AAP coding sequence, wherein the VP3 start codon in the accessory protein coding sequence is silenced.

In some embodiments, the accessory protein coding sequence is located downstream of the replication protein coding sequence. In an alternative embodiment, the accessory protein coding sequence may be located upstream of the replication protein coding sequence. It is understood, that the rep plasmid may also comprise two or more accessory protein coding sequences, wherein these coding sequences may be located upstream and/or downstream of the replication protein coding sequence.

In some embodiments, the rep plasmid comprises a 5'UTR upstream of the accessory protein coding sequence. The rep plasmid can e.g., comprise a 5'UTR immediately upstream of the accessory protein coding sequence, meaning the 5'UTR can be located at the 5' end of the accessory protein coding sequence. Preferably, the 5'UTR is derived from the sequence located between the native p40 promoter and the native accessory protein coding sequence. In some embodiments, the 5' UTR comprises a sequence set forth in SEQ ID NOs: 13 or 14 or a sequence having at least 60%, at least 70%, at least 80% identity, or at least 90% identity to said SEQ ID NOs. In some embodiments, the 5' UTR comprises or consists of a sequence set forth in SEQ ID NOs: 13 or a sequence having at least 60%, at least 70%, at least 80% identity, or at least 90% identity to said SEQ ID NO. In some embodiments, the 5' UTR comprises or consists of a sequence set forth in SEQ ID NO: 14 or a sequence having at least 60%, at least 70%, at least 80% identity, or at least 90% identity to said SEQ ID NO. In some embodiments, the 5'UTR is derived from the sequence located between the native p81 promoter and the native accessory protein coding sequence such as the X coding sequence.

In some embodiments, the rep plasmid comprises a 3'UTR downstream of the accessory protein coding sequence. The rep plasmid may comprise a 3'UTR immediately downstream of the accessory protein coding sequence, meaning the 3'UTR can be located at the 3' end of the accessory protein coding sequence. In some embodiments, the 3'UTR is derived from the sequence located between the native capsid protein coding sequence and the native 3'ITR.

In some embodiments, the 3'UTR comprises one or more RBEs and/or one or more polyadenylation signal sequences. In some embodiments, the 3'UTR comprises one or more RBEs and one or more polyadenylation signal sequences. In some embodiments, the 3'UTR comprises one or more RBEs or one or more polyadenylation signal sequences. In some embodiments, the one or more RBE comprised in the 3'UTR is derived from the p5 promoter. In some embodiments, the one or more RBEs comprised in the 3'UTR consists of the sequence shown in SEQ ID NO: 12.

In some embodiments, the 3'UTR comprises one or more polyadenylation signal sequences. In some embodiments, the one or more polyadenylation signal sequence is selected from a native and/or a heterologous polyadenylation signal sequence. In some embodiments, the one or more polyadenylation signal sequence is selected from a native and a heterologous polyadenylation signal sequence. In some embodiments, the one or more polyadenylation signal sequence is selected from a native or a heterologous polyadenylation signal sequence. In some embodiments, the heterologous polyadenylation signal sequence comprised in the 3' UTR is a heterologous polyadenylation signal sequence. In some embodiments, the heterologous polyadenylation signal sequence comprised in the 3' UTR comprises or consists of the SEQ ID NO: 19 or a sequence having at least 60%, at least 70%, at least 80%, or at least 90% identity to said SEQ ID NO.

In some embodiments, the 3' UTR comprises at least two polyadenylation signal sequences. In some embodiments, the 3' UTR comprises two polyadenylation signal sequences. In such embodiments, the (at least) two polyadenylation signal sequences can either be the same polyadenylation signal sequence or can be different polyadenylation signal sequences. For example, the 3' UTR can comprise a first native polyadenylation signal sequence (i.e., a polyadenylation signal occurring in a wild-type AAV) and a second heterologous polyadenylation signal sequence, e.g., a rabbit β-globin poly(A) signal sequences as shown in SEQ ID NO: 19.

In some embodiments, the 3' UTR comprises a sequence set forth in SEQ ID NOs: 15, 16, or 17 or a sequence having at least 60%, at least 70%, at least 80%, or at least 90% identity to said SEQ ID NO. In some embodiments, the 3' UTR comprises a sequence set forth in SEQ ID NO: 15 or a sequence having at least 60%, at least 70%, at least 80%, or at least 90% identity to said SEQ ID NO. In some embodiments, the 3' UTR comprises a sequence set forth in SEQ ID NO: 16 or a sequence having at least 60%, at least 70%, at least 80%, or at least 90% identity to said SEQ ID NO. In some embodiments, the 3' UTR comprises a sequence set forth in SEQ ID NO: 17 or a sequence having at least 60%, at least 70%, at least 80%, or at least 90% identity to said SEQ ID NO.

In some embodiments, the rep plasmid comprises one or more polyadenylation signal sequences.

In some embodiments, the polyadenylation signal sequence (PAS) is capable of forming a proper poly(A) sequence at the RNA's 3' end. Exemplary polyadenylation signal sequences are an adenovirus L3 poly(A) signal sequence, HSV TK poly(A) signal sequence, hGH poly(A) signal sequence, spA poly(A) signal sequence, rabbit gbpA poly(A) signal sequence, sNRP1 poly(A) signal sequence, bGH poly(A) signal sequence, synthetic poly(A) signal sequence, mouse β-globin poly(A) signal sequence, rabbit β-globin poly(A) signal sequence, H4-based poly(A) signal sequence, and SV40 poly(A) signal sequence. In preferred embodiments, the polyadenylation signal sequence is a SV40 poly(A) signal sequence. In some more preferred embodiments, the polyadenylation signal sequence is a rabbit β-globin poly(A) signal sequence. In some more preferred embodiments, the polyadenylation signal sequence comprises or consists of the SEQ ID NO: 19 or a sequence having at least 60%, at least 70%, at least 80%, or at least 90% identity to said SEQ ID NO.

In some embodiments, the polyadenylation signal sequence is a native polyadenylation signal sequence, i.e., a polyadenylation sequence comprised in the wildtype AAV genome. In some embodiments, the polyadenylation signal sequence is a heterologous polyadenylation signal sequence, i.e., a polyadenylation signal sequence which is not comprised in the wild type AAV genome.

In some embodiments, the polyadenylation signal sequence can be a sequence comprising AATAAA or a modified sequence thereof. Preferably, the polyadenylation signal sequence used in context of the disclosure is a strong polyadenylation signal sequence, which efficiently prompts nuclear export and protein translation such as the SV40 polyadenylation signal. The modified sequence of AATAAA can be a sequence in which one or two nucleic acids are deleted, substituted, inserted and/or added. Other PAS are known and may comprise, for example, ATTAAA, AGTAAA, TATAAA, CATAAA, GATAAA, AATATA, AATACA, AATAGA, AAAAAG, and ACTAAA, and can be used in context of the present disclosure. The polyadenylation signal sequence can be located several nt downstream of the accessory protein coding sequence (e.g., 5, 10, 20, 30, 40, 50 60, 70, 80, 90, 100, 200, 300, 400, 500, 600, 700, or more nt) as known to a skilled person. The polyadenylation signal sequence can be brought into proximity of the accessory protein coding sequence. For clarity, the plasmids disclosed herein can comprise sequences (or elements) that can control or influence polyadenylation such as U/GU-rich sequences, which can be located upstream or downstream of the AATAAA or a modified sequence, e.g., for example separated by several (random) nt (5, 10, 20, 30, 40, 50 60, 70, 80, 90, 100, 1000, 2000, or more nt).

Preferably, the heterologous polyadenylation signal sequence used in context of the disclosure is a strong polyadenylation signal sequence. Without wishing to be bound by theory, a strong polyadenylation signal is contemplated to improve the nuclear export of long and/or short rep protein mRNA, leading to increased translation of long and/or short rep protein mRNA and therefore to higher titers of functional rAAV particles, i.e., rAAV particles with improved potency. The strength of a given polyadenylation signal sequence as described herein refers to the ability of the polyadenylation signal sequence to terminate transcription and initiate polyadenylation of the mRNA's 3' end. Polyadenylation signal sequence strength can be determined based on the degree of polyadenylation of a given mRNA.

The skilled person can therefore readily determine the strength of a polyadenylation signal sequence by using, e.g., PCR-based methods (e.g., digitalPCR, real-time quantitative PCR, RNA-Seq), Northern Blotting, and Microarray methods to determine mRNA polyadenylation levels. Non-limiting examples of heterologous strong polyadenylation signal sequences are SV40 and rabbit β-globin poly(A) signal sequences. In some preferred embodiments, the heterologous strong polyadenylation signal sequence comprises or consists of the SEQ ID NO: 19 or a sequence having at least 60%, at least 70%, at least 80%, or at least 90% identity to said SEQ ID NO. Exemplary methods for determining the polyadenylation are described in Hoque, M., Ji, Z., Zheng, D. et al. Analysis of alternative cleavage and polyadenylation by 3' region extraction and deep sequencing. Nat Methods 10, 133-139 (2013). https://doi.org/10.1038/nmeth.2288 and Proudfoot, Nick J. "Ending the message: poly(A) signals then and now." Genes & development vol. 25,17 (2011): 1770-82. doi:10.1101/gad.17268411.

In some embodiments, the rep plasmid comprises one or more polyadenylation signal sequences. In some embodiments, the rep plasmid comprises at least two polyadenylation signal sequences. In some embodiments, the rep plasmid comprises two polyadenylation signal sequences. In such embodiments, the (at least) two polyadenylation signal sequences can either be the same polyadenylation signal sequence or can be different polyadenylation signal sequences. For example, the rep plasmid can comprise a first native polyadenylation signal sequence and a second strong heterologous polyadenylation signal sequence, e.g., a rabbit β-globin poly(A) signal sequences as shown in SEQ ID NO:19.

### The plasmid system of the invention

In another aspect, the invention provides a plasmid system for producing an adeno-associated virus particle comprising:
(i) a cap plasmid comprising at least one adeno-associated virus capsid protein coding sequence encoding at least one functional capsid protein, and
(ii) the rep plasmid disclosed herein.

Optionally, the plasmid system disclosed herein can be a two-plasmid system comprising all the necessary genetic information for the production of rAAV. For example, the two-plasmid system may comprise a rep plasmid comprising at least one replication protein coding sequence encoding a functional rep protein, a cap plasmid comprising at least one capsid protein coding sequence encoding a functional cap protein, wherein the rep plasmid or the cap plasmid further comprise at least one helper gene. In some embodiments, the rep plasmid or cap plasmid comprises one or more coding sequences encoding for Ela, Elb, E2A, L7 22k, L433k, E4orf6, E4orf7 fusion, or virus-associated RNAs or any combination thereof. The two-plasmid system may comprise at least one replication protein coding sequence encoding a functional rep protein, at least one capsid protein coding sequence encoding a functional cap protein, at least one helper gene and a transgene. In some embodiments, the rep plasmid or cap plasmid comprises a transgene, preferably the cap plasmid comprises the transgene. In some such embodiments, the plasmid system does not comprise a separate transgene plasmid, meaning the plasmid system may not comprise a transgene plasmid in addition to the rep plasmid and the cap plasmid. In some such embodiments, the plasmid system does not comprise a separate helper plasmid, meaning the plasmid system may not comprise a helper plasmid in addition to the rep plasmid and the cap plasmid.

In some embodiments, the plasmid system comprises an additional transgene plasmid and/or an additional helper plasmid. The plasmid system disclosed herein can therefore comprise at least three plasmids:
(i) the cap plasmid comprising at least one adeno-associated virus capsid protein coding sequence encoding at least one functional capsid protein,
(ii) the rep plasmid disclosed herein, and
(iii) a transgene plasmid.

The plasmid system disclosed herein can comprise:
(i) a cap plasmid comprises at least one adeno-associated virus capsid protein coding sequence encoding at least one functional capsid protein,
(ii) the rep plasmid disclosed herein, and
(iii) a helper plasmid.

In some embodiments, the plasmid system further comprises a transgene plasmid. In some embodiments, the plasmid system further comprises a helper plasmid. Typically, AAV can be replication defective and may require co-infection by adenovirus or herpes virus in order to replicate efficiently. The individual adenoviral genes (ordinarily named "helper genes") that contribute to AAV helper function are well-known and have been inter alia identified by testing adenovirus mutants for their ability to mediate AAV replication. The helper plasmid preferably comprises one or more coding sequences encoding for Ela, Elb, E2A, L7 22k, L433k, E4orf6, E4orf7 fusion, or virus-associated RNAs or any combination thereof. These genes (i.e., helper genes) are known to support or participate in AAV replication. In some embodiments, the helper plasmid preferably comprises one or more coding sequences encoding for E2A, L7 22k, L433k, E4orf6, E4orf7 fusion, or virus-associated RNAs or any combination thereof.

When using cells expressing the adenoviral E1a/b genes (such as HEK293T cells) the remaining adenoviral helper genes may encode E4, E2A and virus-associated RNA I and II.

In some embodiments, the helper plasmid comprises one or more virus-associated RNAs. Adenovirus 'virus-associated' RNAs (VA RNAs) are suggested to be abundant, heterogeneous, non-coding RNA transcripts, typically comprising 150-200 nucleotides. For instance, VA RNA I is recognized for its function in relieving the cellular anti-viral blockade of protein synthesis through inhibition of the double-stranded RNA-activated protein kinase (PKR). More recent evidence has revealed that VA RNAs interfere with several other host cell processes.

In some embodiments, the helper plasmid does not comprise the adenoviral late genes. In the context of this disclosure, adenoviral late genes refer to adenovirus genes that are expressed at a later stage in the adenoviral reproduction cycle, i.e., after the expression of the early adenoviral genes, i.e., after the expression of the E1A, E1B, E2A, E2B, E3, and E4 genes. Without wishing to be bound by theory, it is contemplated that the adenoviral late genes encode for adenoviral capsid proteins and are involved in the assembly of adenoviral capsids. In some embodiments, the helper plasmid does not comprise the adenoviral L1-L5 late genes. In some embodiments, the helper plasmid is not able to produce functional adenovirus particles, e.g., adenovirus serotype 5 particles.

The plasmid system disclosed herein can comprise at least four plasmids:
(i) a cap plasmid comprises at least one adeno-associated virus capsid protein coding sequence encoding at least one functional capsid protein,
(ii) the rep plasmid disclosed herein,
(iii) a transgene plasmid, and
(iv) a helper plasmid.

In some embodiments, the transgene is a reporter gene. In preferred embodiments, the reporter gene can be detected by antibody-based assays. In further preferred embodiments, the reporter gene is a fluorescent molecule. Exemplary fluorescent molecules suitable as reporter gene are GFP, eGFP, mGFP, eYFP, citrine, eCFP, mCFP, Cerulean, dtTomato, and any variants thereof. In some embodiments, the reporter gene is a beta-galactosidase, luciferase or glutathione S-transferase, or any variant thereof. The plasmid system disclosed herein is not limited to any particular transgene or reporter gene, and any transgene or reporter gene known in the art can be used.

The transgene can be operatively linked to any suitable promoter. Exemplary suitable promoters can be a chicken β-actin (CBA) promoter, a short CMV early enhancer/chicken β actin (sCAG) promoter, human cytomegalovirus (hCMV) promoter, mouse phosphoglycerate kinase (mPGK) promoter, and human synapsin (hSYN) promoter. In some preferred embodiments, the promoter is a CMVie promoter.

In some embodiments, the transgene has at most 5 kb or 4.7 kb. In some embodiments, the transgene has at most 2.4 kb.

In some embodiments, the plasmid system can comprise two or more transgene plasmids. The first transgene plasmid may comprise a 3' splice donor and the second with a 5' splice acceptor. When both plasmids are expressed in a cell, they can form concatemers, are spliced together, and the full-length transgene can then be expressed. In other embodiments, a transgene is divided between two transgene plasmids, but with substantial sequence overlap. Co-expression can induce homologous recombination and expression of the full-length transgene.

In some embodiments, the transgene plasmid disclosed herein comprises a promoter, a transgene, a polyadenylation signal sequence, 5' and 3' inverted terminal repeats, wherein the transgene plasmid is selected from one of the following:
(i) conventional single-stranded genome recombinant adeno-associated virus, or
(ii) self-complementary genome recombinant adeno-associated virus.

In some embodiments the transgene plasmid is a self-complementary AAV (scAAV) plasmid. Because the conventional ssAAV virus depends on the DNA replication machinery to synthesize the complementary DNA strand, transgene expression may be delayed. To overcome this rate-limiting step, scAAV contains complementary sequences that are capable of spontaneously annealing, upon infection, which eliminates the requirement for host cell DNA synthesis. Methods of generating scAAV plasmids are well known to the person skilled in the art. In some embodiments, the scAAV plasmid comprises a transgene of about 2.4 kb or less.

In some embodiments, at least one inverted terminal repeats, e.g., the 5' inverted terminal repeats, has a deletion compared to the native inverted terminal repeats. In some embodiments, the transgene plasmid comprising the 5'ITR deletion is a scAAV plasmid.

In some embodiments, the 3' and 5' inverted terminal repeats are independently derived from an ITR sequence of any AAV serotype, including but not limited to, AAV type 1 (e.g., AAV of serotype 1, also referred to as AAV1), AAV type2 (e.g., AAV2), AAV type 3 (e.g., AAV3, including types 3A and 3B, AAV3A and AAV3B), AAV type 4 (e.g., AAV4), AAV type 5 (e.g., AAV5), AAV type 6 (e.g., AAV 6), AAV type 7 (e.g., AAV7), AAV type 8 (e.g., AAV8), AAV type 9 (e.g., AAV9), AAV type 10 (e.g., AAV10), AAV type 11 (e.g., AAV 11), AAV type 12 (e.g., AAV 12), AAV type 13 (e.g., AAV 13), AAV type rh32.33 (e.g., AAVrh32.33), AAV type rh8 (e.g., AAVrh8), AAV type rhlO (e.g., AAVrhlO), AAV type rh74 (e.g., AAVrh74), AAV type hu.68 (e.g., AAVhu.68), avian AAV (e.g., AAAV), bovine AAV (e.g., BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In preferred embodiments, the 3' and 5' inverted terminal repeats are derived from J01901.1 AAV2.

In some embodiments, the capsid protein coding sequence is from an AVV serotype selected from the group consisting of AAV-1, AAV-2, AAV-3, AAV-4, AAV-5, AAV-6, AAV-7 and AAV-8, preferably selected from AAV2.

In some embodiments, the capsid protein coding sequence encodes one or more capsid proteins selected from VP1, VP2, or VP3 or an artificial variant thereof. In some embodiments, the cap protein sequence comprises or consists of a sequence shown in any of SEQ ID NO: 5-7 or a sequence having at least 60%, at least 70%, at least 80%, or at least 90% identity to said SEQ ID Nos. In some embodiments, the VP1 protein comprises or consists of the SEQ ID NO: 5 or a sequence having at least 60%, at least 70%, at least 80%, or at least 90% identity to said SEQ ID NO. In some embodiments, the VP2 protein comprises or consists of the SEQ ID NO: 6 or a sequence having at least 60%, at least 70%, at least 80%, or at least 90% identity to said SEQ ID NO. In some embodiments, the VP3 protein comprises or consists of the SEQ ID NO: 7 or a sequence having at least 60%, at least 70%, at least 80%, or at least 90% identity to said SEQ ID NO.

In some embodiments, the plasmid system is used for rAAV production, wherein the rAAV can be used in gene therapy applications.

The embodiments disclosed herein for the rep plasmid of the invention can be used for the plasmid system for producing an adeno-associated virus particle as disclosed herein. Features described herein in more detail in connection with the "rep plasmid comprising" embodiments equally apply to the corresponding plasmid system for producing an adeno-associated virus particle embodiments.

### The stable or transient cell expression system and the cell of the invention

In another aspect, the invention provides a stable or transient cell expression system comprising the plasmid system disclosed herein and a cell line.

According to preferred embodiments, the stable expression system comprises the genetic elements of the plasmid system or the rep plasmid disclosed herein. For example, genome editing may be used to stably integrating one or more sequences present on the plasmid(s) or plasmid system disclosed herein into the chromosome of a cell to obtain a cell capable of stably producing a rAAV particle. The stable integration is advantageous in that it allows using the same stock of genome edited cells multiple times without the need to genetically modifying the cells before culturing the cells to produce rAAV particles. The integration can be performed by providing at least one site-directed endonuclease, preferably being selected from a meganuclease, a ZFN, a TALEN, a CRISPR-nuclease, a nickase, or nuclease-dead variant therefrom. The integration can be performed by providing at least one nucleic acid molecule encoding a site-directed endonuclease, preferably being selected from a meganuclease, a ZFN, a TALEN, a CRISPR-nuclease, a nickase, or nuclease-dead variant therefrom. CRISPR gene editing is well known to a skilled person. For example, CRISPR-directed gene integration can be conducted by providing at least one suitable, functional guide RNA molecule, or a nucleic acid molecule encoding the same, and genetic elements required for producing the rAAV in form of a template to be integrated into the genome. The template may be cleaved by the at least one site-directed endonuclease, e.g., a CRISPR-nuclease. The way of integrating the genetic elements into the genome of the host cell shall not be limited and different techniques are known to and available to the skilled person. According to particular embodiments, however, the one or more genetic elements required for viral vector production may be integrated using a CRISPR-nuclease, e.g., Cas12a or Cas9.

In some embodiments, the stable or transient cell expression system comprises the rep plasmid disclosed herein. In this context it is understood that the stable cell expression system does not comprise the plasmid as such but the respective gene(s) of the plasmid, e.g., sequences of a plasmid stably integrated into the genome of the cell. In some embodiments, the stable or transient cell expression system comprises the plasmid system disclosed herein. In some embodiments, the stable or transient cell expression system comprises the rep plasmid disclosed herein and a cap plasmid. In some embodiments, the stable or transient cell expression system comprises the rep plasmid of the invention, a cap plasmid, and a helper plasmid. In some embodiments, the stable or transient cell expression system comprises the rep plasmid of the invention, a cap plasmid, a helper plasmid, and a transgene plasmid. In some embodiments, the stable or transient cell expression system comprises the rep plasmid disclosed herein and a helper plasmid.

In some embodiments, a cell expression system is provided wherein the sequence comprising the rep coding sequence is stably integrated. The cell expression system disclosed herein can provide a stable rep expression system and provide a transient cap, helper, and transgene expression system.

In another aspect, the invention provides a cell comprising the rep plasmid disclosed herein or the plasmid system disclosed herein.

In some embodiments, the cell comprises the rep plasmid disclosed herein and a transgene plasmid. In some embodiments, the cell comprises the rep plasmid disclosed herein. In some embodiments, the cell comprises the plasmid system disclosed herein. In some embodiments, the cell comprises the rep plasmid of the invention and a cap plasmid. In some embodiments, the cell comprises the rep plasmid of the invention, a cap plasmid, and a helper plasmid. In some embodiments, the cell comprises the rep plasmid of the invention and a helper plasmid. In some embodiments, the cell comprises the rep plasmid of the invention, the cap plasmid, the helper plasmid, and a transgene plasmid.

In preferred embodiments, the cell is a mammalian cell as described below. The cells can be HEK293 cells, HELA cells or insect cells, or derivatives. Most preferably, the cell is a HEK293 cell.

### The kit of the invention

In another aspect, the invention provides a kit comprising a stable or transient cell expression system disclosed herein, or a cell disclosed herein, and a cell culture medium.

In some embodiments, the kit comprises media feeds, media additives or transfection reagents or any combination thereof. In some embodiments, the kit comprises a manual.

### The method of producing recombinant adeno-associated viral vectors of the invention

In another aspect, the invention provides a method of producing recombinant adeno-associated viral vectors comprising:
(i) transfecting cells with the rep plasmid disclosed herein, or the plasmid system disclosed herein;
(ii) culturing the transfected cells to produce said adeno-associated viral vectors; and
(iii) isolating said recombinant adeno-associated viral vectors.

In some embodiments, the cells are HEK293 cells, HELA cells, CHO cells, insect cells, or derivatives thereof. For example, the insect cells can be SF9 cells.

In some embodiments, the cells are mammalian cells. A mammalian cell is a cell that is of mammalian origin. However, the cell does not need to be identical to a cell obtained in a mammalian but can be modified, engineered or naturally/artificially changed. For instance, the mammalian cell may be a cell with mammalian origin, however, may contain one or more genetic changes to propagate it repeatedly and possibly also infinitely. The term "mammalian cell" may be interchangeable used with "mammalian cells" and shall cover both the singular and plural form. The mammalian cell is preferably a mammalian cell line. According to a preferred embodiment, the mammalian cell is a human-derived cell line, which advantageously has the ability to provide the suitable post-translational modifications for therapy in humans. This means that the produced viral vector has similar post-translational modification, as if the native virus would have infected a human cell resulting in virus production.

The mammalian cell can be selected from the group consisting of HeLa, Human embryonic kidney 293 (HEK293), BSC-1, SW480, Baby hamster kidney (BHK), BHK-21, Vero E6, U2OS, A549, HT1080, CAD, P19, NIH 3T3, L929, N2a, Chinese hamster ovary (CHO), MCF-7, Y79, SO-Rb50, Hep G2, DUKX-X11, J558L, HuH-7, MDCK, or HepG2 cells or derivatives thereof. According to a preferred embodiment, the mammalian cell is selected from the group consisting of HEK293, A549, BSC-1, SW480, Baby hamster kidney (BHK), Vero E6 and MDCK cells or a derivative thereof.

While "HEK293" are established in the field for viral vector production, since their creation in the 1970ies various derivatives thereof have been generated, which are also well suited for viral vector production. In general, according to preferred embodiments, the derivative of the HEK293 cell is characterized by being cell that roots back to HEK293 and/or was originally a HEK293 cell that was adapted, modified, differentiated, reprogrammed, transformed, transduced, transfected and/or mutated. The skilled person readily can identify a HEK293 cell as known in the art, as it typically contains the adenovirus 5 DNA inserted in human chromosome 19. While it is not excluded that such adenovirus 5 DNA insert has been modified, it is believed that certain similarities will always be existent in order to identify a HEK293 cell or a respective derivative thereof. According to preferred embodiments, the derivative of the HEK293 cell is selected from one or more of the group consisting of 293, 293T, 293T/17, ANJOU 65, 293H, 293E, 293-6E, EBNA1-6E, 293F, 293FT, 293Flp-IN T-REx, 293FTM, 293 S, 293SG, 293SGGD, 293MSR, 293A, or any modified variants thereof. Such derivatives may be advantageous for viral vector production. Some of these derivatives may have been particularly adapted in the past in order to enhance viral vector production.

In some aspects, the invention provides a rAAV preparation obtainable by the method disclosed herein.

### SEQUENCE LISTING

This application contains a Sequence Listing which has been submitted electronically and is hereby incorporated by reference in its entirety. Said Sequence Listing file is named 240384EP_Sequence Listing.XML and 32.884 Bytes in size.

SEQ ID NOs: 1, 2, 3, and 4 are exemplary amino acid sequences of the rep78, rep68, rep52, and rep40 proteins, respectively.

SEQ ID NOs: 5, 6, and 7 are exemplary amino acid sequences of the VP1, VP2, and VP3 proteins, respectively.

SEQ ID NOs: 8, 9, and 10 are exemplary amino acid sequences of the AAP, MAAP, and X proteins, respectively.

SEQ ID NO: 11 is an exemplary amino acid sequence of an eGFP fluorescent reporter suitable for use as transgene of the disclosure.

SEQ ID NO: 12 is an exemplary polynucleotide sequence comprising or consisting of a rep-binding element (RBE) derived from the p5 promoter.

SEQ ID NOs: 13 and 14 are exemplary polynucleotide sequences of accessory protein 5'UTRs.

SEQ ID NOs: 15, 16, 17, and 22 are exemplary polynucleotide sequences of accessory protein and capsid protein 3'UTRs.

SEQ ID NO: 18 is an exemplary polynucleotide sequence comprising or consisting of a rep-binding element (RBE) derived from an inverted terminal repeats sequence.

SEQ ID NO: 19 is an exemplary polynucleotide sequence of a rabbit β-globin poly(A) signal sequence.

SEQ ID NO: 20 exemplary polynucleotide sequences of a capsid protein 3' UTR.

SEQ ID NO: 21 is an exemplary AAV2 complete genome.

### EXAMPLES

### Example 1:

AAVMax cells (ThermoFisher Scientific) were grown in HEK ViP NB medium (Sartorius Xell) in an Ambr15^{®} cell culture bioreactor for four days at a DO of 40%, a rpm of 630, and a pH range of 7.0 - 7.2. Cell densities were maintained between 0.3 and 4 million viable cells/mL cell culture. On the fifth day, the cells were transfected with rep-cap plasmids or rep-cap split plasmids, helper plasmids, and EGFP transgene plasmids at 1µg/3.5-4 million viable cells/mL using PEIPro^{®} as the transfection reagent. The molar ratios of transfected plasmids were 1:1:1 (3 plasmid system, e.g., rep-cap split plasmids: helper plasmids: EGFP transgene-cap plasmids) and the volume of the transfection mixture was 15% v/v final volume. For the rep-cap split plasmids, the respective missing cap and accessory proteins were provided in trans on the EGFP transgene plasmids. Twenty-four hours post transfection, the cells were fed by addition of 10% v/v FS medium (Sartorius Xell). After 72 hours, cells were lysed and benzonase digested and potency (TU/L) and viral genome titers (vg/L) were determined. Potency was determined based on eGFP transgene expression in the target cell using the Incucyte^{®} Live-Cell Analysis System and TU/L crude lysate assessment by Poisson distribution. Viral genome titers were determined by ddPCR.

The following rep-cap and rep-cap split plasmids were investigated:
- Plasmid NO: 1 (Rep-Cap (RBE)) comprising:
   ∘ wild-type rep and cap genes, thus comprising the coding sequences for the long rep proteins rep78/68 (SEQ ID NO: 1 and 2) and short rep proteins rep 52/40 (SEQ ID NO: 3 and 4), and coding sequences for the cap proteins V1, VP2, and VP3 (SEQ ID NO: 5, 6 and 7), driven by their native promoters;
   ∘ coding sequences for the accessory proteins MAAP, AAP, and X (SEQ ID NO: 8, 9, and 10) driven by their native promoter;
   ∘ a 3' UTR comprising a (weak) AAV polyA signal (SEQ ID NO: 22), and an RBE derived from a 3' inverted terminal repeat (SEQ ID NO: 18);
- Plasmid NO: 2 (Rep-Cap (pMMTV + p5RBE + polyA)) comprising:
   ∘ coding sequences for the long rep proteins rep78/68 (SEQ ID NO: 1 and 2) operatively linked to the MMTV promoter;
   ∘ coding sequences for the short rep proteins rep 52/40 (SEQ ID NO: 3 and 4) driven by their native promoter,
   ∘ coding sequences for the cap proteins V1, VP2, and VP3 (SEQ ID NO: 5, 6 and 7) driven by their native promoter;
   ∘ coding sequences for the accessory proteins MAAP, AAP, and X (SEQ ID NO: 8, 9, and 10) driven by their native promoter;
   ∘ a 3' UTR comprising a (weak) AAV polyA signal (SEQ ID NO: 22), a p5-derived RBE (SEQ ID NO: 12), and a rabbit beta-globin polyA signal (SEQ ID NO:19).
- Plasmid NO: 3 (Rep-Cap split (pMMTV + p5RBE + polyA + p40_si)) comprising:
   ∘ coding sequences for the long rep proteins rep78/68 (SEQ ID NO: 1 and 2) operatively linked to the MMTV promoter;
   ∘ coding sequences for the short rep proteins rep 52/40 (SEQ ID NO: 3 and 4) driven by their native promoter;
   ∘ a silenced p40 promoter (p40_si) by codon optimization and no coding sequences for cap proteins and accessory proteins;
   ∘ a 3' UTR comprising a (weak) AAV polyA signal (SEQ ID NO: 20), a p5-derived RBE (SEQ ID NO: 12), and a rabbit beta-globin polyA signal (SEQ ID NO:19).
- Plasmid NO: 4 (Rep-Cap split (p40_si)) comprising:
   ∘ coding sequences for the long rep proteins rep78/68 (SEQ ID NO: 1 and 2) and short rep proteins rep 52/40 (SEQ ID NO: 3 and 4) driven by their native promoters;
   ∘ a silenced p40 promoter (p40_si) and no coding sequences for cap proteins and accessory proteins; and
   ∘ a 3' UTR comprising a AAV weak polyA signal (SEQ ID NO: 20).

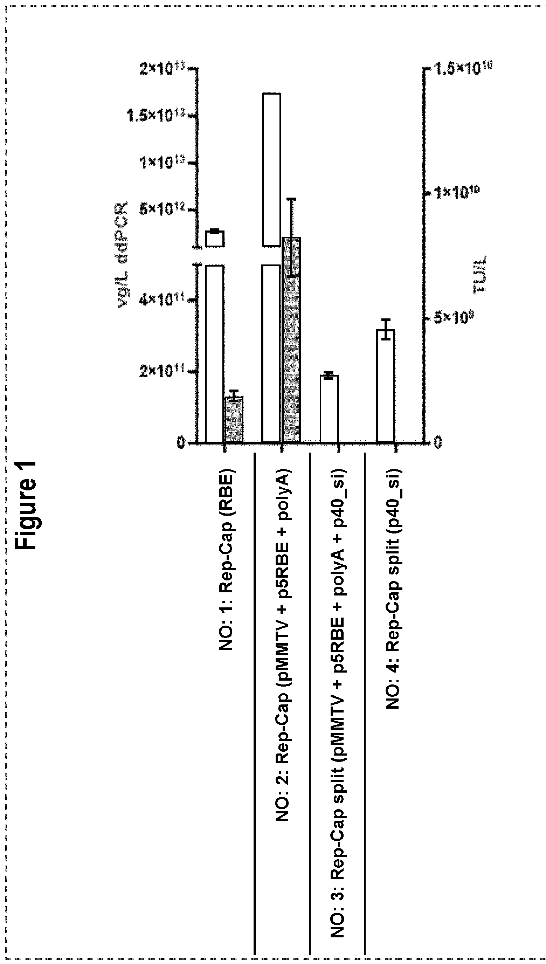

Figure 1 shows that the viral genome titers and the potency are significantly reduced for rAAVs obtained by a rep-cap split plasmid (cf. plasmids NO: 3 and 4) compared to rep-cap plasmids (cf. plasmids NO: 1 and 2).

### Example 2:

AAVMax cells (ThermoFisher Scientific) were grown in HEK ViP NB medium (Sartorius Xell) in an Ambr15^{®} cell culture bioreactor for four days at a DO of 4%0, a rpm of 630, and a pH range of 7.0 - 7.2. Cell densities were maintained between 0.5 and 4 million viable cells/mL cell culture. On the fifth day, the cells were transfected with rep-cap split plasmids, helper plasmids, and EGFP transgene plasmids at a density of 3.5-4 million viable cells/mL at 1 µg of plasmid per million viable cells using PEIPro^{®} as the transfection reagent. The molar ratios of transfected plasmids were 1:1:1 (3 plasmid system, e.g., rep-cap split plasmids: helper plasmids: EGFP transgene-cap plasmids) and the volume of the transfection mixture was 15% v/v final volume. The respective missing cap and accessory proteins were provided in trans on the EGFP transgene plasmids. Twenty-four hours post transfection, the cells were fed by addition of 10% v/v FS medium (Sartorius Xell). After 72 hours, cells were lysed and benzonase digested and potency (TU/L) and viral genome titers (vg/L) were determined. Potency was determined based on eGFP transgene expression in the target cell using the Incucyte^{®} Live-Cell Analysis System and TU/L crude lysate assessment by Poisson distribution. Viral genome titers were determined by ddPCR.

The following rep-cap split plasmids were investigated:
- Plasmid NO: 5 (Rep-Cap split (RBE + p40_si + cap_si)) comprising:
   o wild-type gene, thus comprising the coding sequences for the long rep proteins rep78/68 (SEQ ID NO: 1 and 2) and short rep proteins rep 52/40 (SEQ ID NO: 3 and 4) driven by their native promoters;
   o a silenced p40 promoter (p40_si);
   o silenced cap proteins through mutated VP1, VP2, VP3 start codons (cap_si);
   o silenced accessory proteins through mutated MAAP, AAP, and X start codons;
   o a 3' UTR; and
   o an RBE derived from a 3' inverted terminal repeat (SEQ ID NO: 18).
- Plasmid NO: 6 (Rep-Cap split (pMMTV + p40 MAAP + p5RBE + polyA)) comprising:
   o coding sequences for the long rep proteins rep78/68 (SEQ ID NO: 1 and 2) operatively linked to the MMTV promoter;
   o coding sequences for the short rep proteins rep 52/40 (SEQ ID NO: 3 and 4) driven by their native promoter;
   o coding sequence for MAAP (SEQ ID NO: 9) comprising 2 stop codons and driven by its native promoter p40;
   o a silenced VP1 start codon, and no coding sequences for the AAP, and X proteins;
   o a truncated VP1 coding sequence due to the overlap with the MAAP coding sequence;
   o a 3' UTR comprising a (weak) AAV polyA signal, a p5-derived RBE (SEQ ID NO: 12), and a rabbit beta-globin polyA signal (SEQ ID NO: 19).
- Plasmid NO: 7 (Rep-Cap split (pMMTV + p40 AAP + p5RBE + polyA)) comprising:
   o coding sequences for the long rep proteins rep78/68 (SEQ ID NO: 1 and 2) operatively linked to the MMTV promoter;
   o coding sequences for the short rep proteins rep 52/40 (SEQ ID NO: 3 and 4) driven by their native promoter;
   o coding sequence for AAP (SEQ ID NO: 9) comprising 2 stop codons and driven by its native promoter p40;
   o silenced VP1 and VP3 start codons, and no coding sequences for the MAAP, and X proteins;
   o a truncated VP1, VP2, and VP3 coding sequence due to the overlap with the AAP coding sequence;
   o a 3' UTR comprising a (weak) AAV polyA signal, a p5-derived RBE (SEQ ID NO: 12), and a rabbit beta-globin polyA signal (SEQ ID NO: 19).
- Plasmid NO: 8 (Rep-Cap split (pMMTV + p40 MAAP/AAP + p5RBE + polyA)): comprising:
   o coding sequences for the long rep proteins rep78/68 (SEQ ID NO: 1 and 2) operatively linked to the MMTV promoter;
   o coding sequences for the short rep proteins rep 52/40 (SEQ ID NO: 3 and 4) driven by their native promoter;
   o coding sequence for AAP and MAAP (SEQ ID NO: 8 and 9) each comprising 2 stop codons and driven by its native promoter p40;
   ∘ silenced VP1, VP2, and VP3 start codons, and no coding sequences for the X protein;
   ∘ a truncated VP1, VP2, and VP3 coding sequence due to the overlap with the AAP and MAAP coding sequence;
   ∘ a 3' UTR comprising a (weak) AAV polyA signal, a p5-derived RBE (SEQ ID NO: 12), and a rabbit beta-globin polyA signal (SEQ ID NO: 12).
- Plasmid NO: 4 (Rep-Cap split (p40_si)) as described above.

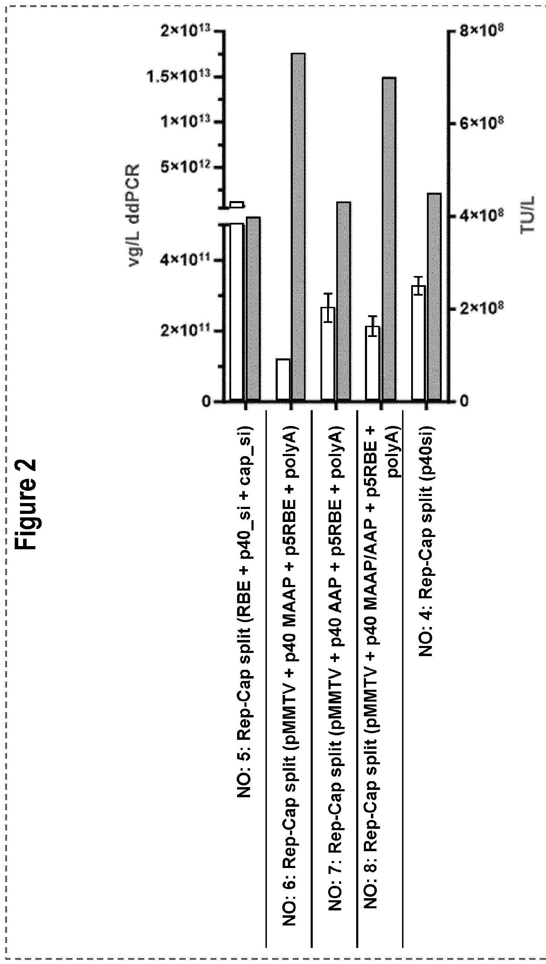

Figure 2 shows that the measured potency substantially improved for the rep-cap split plasmids comprising the accessory protein coding sequence, in particular the MAAP coding sequence, in cis to the rep coding sequences. This demonstrates that the rep plasmid of the invention can be advantageously used in an rAAV production process, since the potency of the rAAV vectors obtained by the rep-plasmid of the invention is improved compared to conventional rep-cap split plasmid.

### BRIEF DESCRIPTION OF THE DRAWINGS

Fig 1. depicts the measured concentrations of viral genomes [vg/L] and transducing titers [TU/L] of different rep-cap and rep-cap split plasmids (plasmids NO: 1, 2, 3, and 4).
Fig 2. depicts the measured concentration of viral genomes [vg/L] and transducing titers [TU/L] of different rep-cap split plasmids (plasmids NO: 4, 5, 6, 7, and 8).

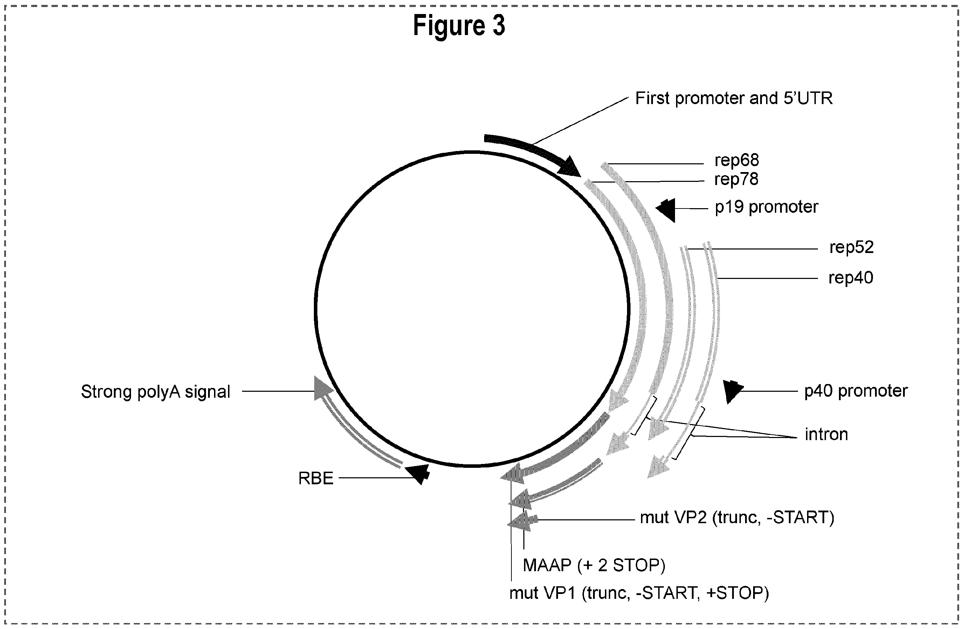

Fig 3. depicts a schematic representation of an exemplary embodiment of the rep plasmid of the invention. The rep plasmid depicted in Figure 3 comprises a first promoter, long rep protein (rep68/78) and short rep protein (rep40/52) coding sequences, a MAAP coding sequence comprising a silenced VP1 and VP2 start codon, a heterologous polyadenylation signal sequence (strong polyA signal), and p19 and p40 promoters. The first promoter regulates the long rep protein coding sequences and the p40 promoter regulates the transcription of the MAAP coding sequences. The VP1 and VP2 coding sequences are truncated (trunc) due to their overlap with the MAAP coding sequence. The MAAP coding sequence comprises two stop codons at the 3' end (+2 STOP). The schematic representation comprises an RBE downstream of the MAAP coding sequence and upstream the polyadenylation signal sequence.

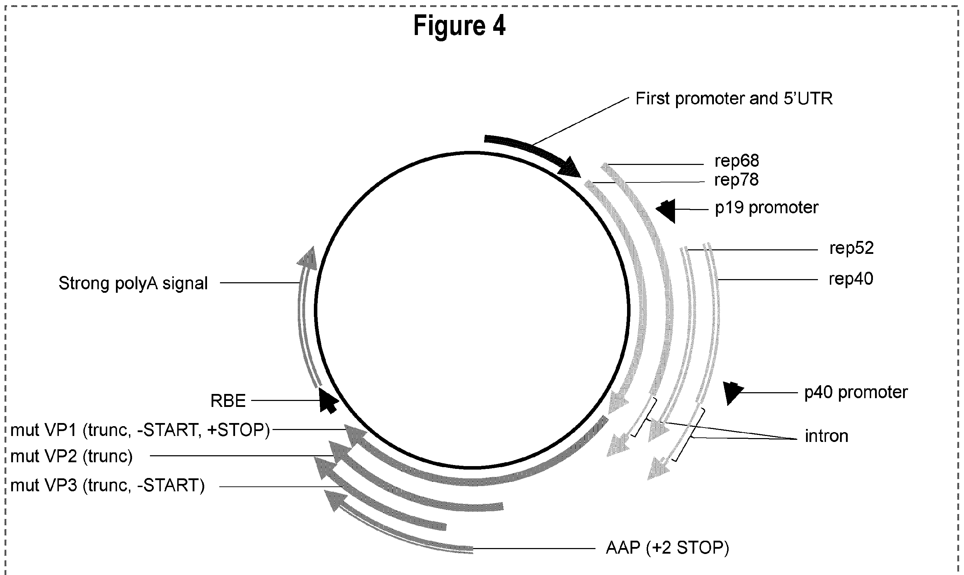

Fig 4. depicts a schematic representation of an exemplary embodiment of the rep plasmid of the invention. The rep plasmid depicted in Figure 4 comprises a first promoter, long rep protein (rep68/78) and short rep protein (rep40/52) coding sequences, an AAP coding sequence, a heterologous polyadenylation signal sequence (strong polyA signal), and p19 and p40 promoters. The first promoter regulates the long rep protein coding sequences and the p40 promoter regulates the transcription of the AAP coding sequence. The VP1, VP2, and VP3 coding sequences are truncated (trunc) and the VP1 and VP3 coding sequences comprise a silenced start codon. The AAP coding sequence comprises two stop codons at the 3' end (2+ STOP). The schematic representation comprises a RBE downstream of the AAP coding sequence and upstream the polyadenylation signal sequence.

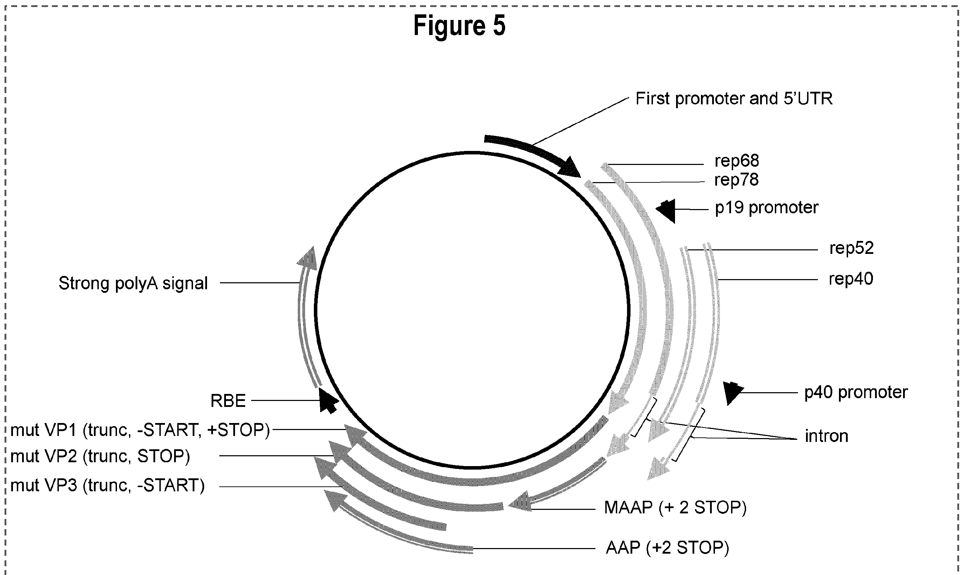

Fig 5. depicts a schematic representation of an exemplary embodiment of the rep plasmid of the invention. The rep plasmid depicted in Figure 5 comprises a first promoter, long rep protein (rep68/78) and short rep protein (rep40/52) coding sequences, MAAP and AAP coding sequences, a heterologous polyadenylation signal sequence (strong polyA signal), and p19 and p40 promoters. The first promoter regulates the long rep protein coding sequences and the p40 promoter regulates the transcription of the MAAP and AAP coding sequences. The VP1, VP2, and VP3 coding sequences are truncated (trunc) and the VP1 and VP2 coding sequences comprise a silenced start codon. The MAAP and AAP coding sequences comprise two stop codons at the 3' end (+ 2 STOP). The schematic representation comprises a RBE downstream of the MAAP and AAP coding sequences and upstream the polyadenylation signal sequence.

## Claims

1. A rep plasmid comprising:
(i) at least one adeno-associated virus replication protein coding sequence encoding at least one functional rep protein; and
(ii) at least one accessory protein coding sequence;
wherein the rep plasmid is not capable of encoding a functional cap protein.

2. The rep plasmid according to claim 1, wherein the rep plasmid comprises at least one replication protein coding sequence encoding at least two functional rep proteins, at least three functional rep proteins, or at least four functional rep proteins.

3. The rep plasmid according to any one of claims 1 or 2, wherein the accessory protein coding sequence encodes one or more of AAP, MAAP, and X, or artificial variants thereof, preferably wherein the accessory protein coding sequence encodes at least MAAP or artificial variants thereof.

4. The rep plasmid according to any one of claims 1-3, wherein the rep plasmid comprises a first accessory protein coding sequence encoding MAAP or an artificial variant thereof and a second accessory protein coding sequence encoding X or an artificial variant thereof.

5. The rep plasmid according to any one of claims 1-4, wherein the rep plasmid does not comprise a capsid protein sequence encoding any functional VP1, VP2, and VP3, or artificial variants thereof.

6. The rep plasmid according to any one of claims 1-5, wherein the rep plasmid comprises a capsid protein sequence encoding a non-functional capsid protein sequence.

7. The rep plasmid according to any one of claims 1-6, wherein the rep plasmid comprises a capsid coding sequence comprising a silenced start codon.

8. A plasmid system for producing an adeno-associated virus particle comprising:
(i) a cap plasmid comprising at least one adeno-associated virus capsid protein coding sequence encoding at least one functional capsid protein, and
(ii) the rep plasmid of any one of claims 1 to 7.

9. The plasmid system according to claim 8, wherein the cap plasmid or the rep plasmid comprises a transgene.

10. The plasmid system according to claim 8, further comprising a transgene plasmid.

11. The plasmid system according to any one of claims 8-10, wherein the system further comprises a helper plasmid, wherein the helper plasmid preferably comprises one or more coding sequences encoding for Ela, Elb, E2A, L7 22k, L433k, E4orf6, E4orf7 fusion, or virus-associated RNAs or any combination thereof.

12. A stable or transient cell expression system comprising the plasmid system according to any one of claims 8-11 and a cell, preferably a cell line.

13. A cell comprising the rep plasmid according to any of claims 1-7 or the plasmid system according to any one of claims 8-11.

14. A kit comprising a stable or transient cell expression system according to claim 12, or a cell according to claim 13, and a cell culture medium.

15. A method of producing recombinant adeno-associated viral vectors comprising:
(i) transfecting cells with the rep plasmid according to any of claims 1-7, or the plasmid system according to any one of claims 8-11;
(ii) culturing the transfected cells to produce said adeno-associated viral vectors; and
(iii) isolating said recombinant adeno-associated viral vectors.
